# EUROPEAN PATENT SPECIFICATION

(11) **EP 1 981 522 B1**
(45) Date of publication and mention of the grant of the patent: **12.09.2012**
(21) Application number: 07763653.8
(22) Date of filing: 23.01.2007
(51) Int. Cl.: C07K 14/705, A61K 38/17, A61K 39/395, A61K 31/7088, A61P 25/00

(54) **COMPOSITIONS AND METHODS FOR ENHANCING NEURONAL PLASTICITY AND REGENERATION**
ZUSAMMENSETZUNGEN UND VERFAHREN ZUR INTENSIVIERUNG DER NEURONALEN PLASTIZITÄT UND REGENERIERUNG
COMPOSITIONS ET PROCEDES DESTINES A ACCROITRE LA PLASTICITE ET LA REGENERATION NEURONALES

(30) Priority: 23.01.2006 US 761453 P; 17.08.2006 US 838583 P; 15.11.2006 US 859372 P
(43) Date of publication of application: 22.10.2008
(73) Proprietor: President and Fellows of Harvard College, Cambridge, MA 02138 (US)
(72) Inventor: SHATZ, Carla, Boston, MA 02110 (US); SYKEN, Joshua, Boston, MA 02115 (US)
(74) Representative: Smart, Peter John
(86) International application number: PCT/US2007/002009
(87) International publication number: WO 2007/092165

(56) References cited:
- WO-A2-03/051834
- WO-A2-2008/061019
- TAKAI TOSHIYUKI: "A novel recognition system for MHC class I molecules constituted by PIR." ADVANCES IN IMMUNOLOGY 2005 LNKD- PUBMED:16227090, vol. 88, 2005, pages 161-192, XP008122314 ISSN: 1557-8445
- BOULANGER LISA M ET AL: "Immune signalling in neural development, synaptic plasticity and disease" NATURE REVIEWS NEUROSCIENCE, vol. 5, no. 7, July 2004 (2004-07), pages 521-531, XP008122246 ISSN: 1471-003X
- SYKEN JOSH ET AL: "PirB restricts ocular-dominance plasticity in visual cortex" SCIENCE, AMERICAN ASSOCIATION FOR THE ADVANCEMENT OF SCIENCE, WASHINGTON, DC; US LNKD- DOI:10.1126/SCIENCE.1128232, vol. 313, no. 5794, 17 August 2006 (2006-08-17), pages 1795-1800, XP002540805 ISSN: 0036-8075 [retrieved on 2006-08-17]
- ATWAL JASVINDER K ET AL: "PirB is a functional receptor for myelin inhibitors of axonal regeneration" SCIENCE, AMERICAN ASSOCIATION FOR THE ADVANCEMENT OF SCIENCE, WASHINGTON, DC; US LNKD- DOI:10.1126/SCIENCE.1161151, vol. 322, no. 5903, 7 November 2008 (2008-11-07), pages 967-970, XP002546231 ISSN: 0036-8075

## Description

### Field of the Invention

The field of the invention relates to the role of the Major Histocompatability Complex Class I molecules, in particular paired immunoglobulin-like receptor-B, in non-immune system related diseases/disorders not mediated by classical immunity and the development of beneficial therapeutic/diagnostic utilities.

### Background of the Invention

Several publications and patent documents are cited throughout the specification in order to describe the state of the art to which this invention pertains. Full citations for those references that are numbered can be found at the end of the specification.

The MHC family is comprised of dozens of genes, some of which are among the most polymorphic loci in the genome. The mouse MHC may be divided into three broad categories: class I (HLA A, B, and C in humans); class II, (HLA DP, DQ, and DR in humans); and class III, which includes components of the compliment system. While MHC class I is well known for its so-called "classical" class I products, which are crucial for the adaptive immune response mediated by T-cells, the majority of class I genes actually encode "nonclassical" MHC class I products, many of which have no known function in the immune system. Some nonclassical class I proteins associate with the MHC class I light chain, β2 microglobulin (β2m), bind and certain peptides, and have high sequence and structural homology with members of the classical MHC class I, although unlike the latter, they display more restricted expression patterns and little or no polymorphism. Interestingly, a recent study located one family of these "orphan" MHC class I's—whose sequence was known, but whose products had not yet been located— exclusively within the vomeronasal organ (VNO), a small pit in the anterior nasal cavity of some mammals that is specialized to detect pheromone.

Recent results suggest that normal, uninjured neurons express both classical and nonclassical MHC class I *in vivo.* MHC class I mRNA and/or protein has been detected in diverse neuronal populations, including motor nuclei, substantia nigra pars compacta, dorsal root ganglia neurons, dopaminergic nigral cells, developing and adult hippocampal pyramidal cells, sensory neurons of the vomeronasal organ, brainstem and spinal motoneurons, and cortical pyramidal cells. *In situ* hybridization with probes specific for individual classical and nonclassical MHC class I genes reveals a complex pattern of MHC class I mRNA expression in the healthy adult brain. Some of these studies also confirm that MHC class I expression in neurons can be further increased by treatments including axotomy, exposure to cytokines, and changes in electrical activity.

MHC class I genes display overlapping but distinct neuronal expression patterns, and these patterns are particularly dynamic during normal development. Along with the fact that MHC class I expression can be regulated by naturally occurring electrical activity, these results suggest that the precise timing and level of MHC class I expression is critical for its function in the brain.

Pioneering studies into MHC class I function in the brain have been initiated based on the identification of members of the MHC class I family in genomic screens of specific neuronal populations. The first hint of a non-immune function for MHC class I in neurons came when it was identified in an unbiased functional screen for genes involved in activity-dependent plasticity in the developing visual system. MHC class I expression was found to decrease after activity blockade with TTX, specifically during the period when spontaneous retinal activity is needed for synaptic refinement of overlapping eye-specific inputs to LGN neurons to form a mature, segregated pattern of connections. Subsequent examination revealed that MHC class I expression closely parallels times and places of activity-dependent plasticity in the developing and adult mammalian brain, including the early postnatal retina and LGN, and adult cerebellum and hippocampus. Together, these observations suggest that MHC class I might be involved in activity-dependent structural and functional plasticity.

Plasticity of connections in the nervous system is thought to be driven by cellular processes that strengthen or weaken existing synapses in response to neuronal activity, followed by long term changes that result in structural alterations to the circuit. The cellular and molecular machinery responsible for synaptic plasticity are well studied. However, the mechanisms and molecular components that couple short term synaptic changes to long term structural remodeling are less well known.

In the immune system, MHCI proteins function through interactions with a variety of transmembrane receptors on immune system cells (4-8). These cell-cell interactions are the means by which normal cells are distinguished from abnormal or foreign cells. In the nervous system, the mechanisms by which neuronal MHCI modulates synaptic development are not understood. One hypothesis that draws inspiration from many examples in the immune system suggests that neuronal MHCI regulates neuronal function by engaging transmembrane MHCI receptors expressed on other neurons. These interactions could generate intracellular signals that ultimately alter synaptic strength, neuronal morphology and circuit properties. Thus, identifying neuronal MHCI receptors may be crucial to understanding mechanisms underlying the neuronal activities of these proteins and the neuronal plasticity that they regulate.

An important early step in understanding the role of MHC class I in the brain is to determine which of the many MHC class I proteins are expressed in neurons, and to characterize the specific expression profile of each MHC class I product in the developing and adult brain. Furthermore, since members of the large MHC Class I gene family are expressed on the surface of most nucleated cells, it would be of major benefit if MHC Class I molecules involved in normal development, disease or response to injury of all cell types could be identified and used as tools for developing therapeutic agents and diagnostic methods.

Boulanger et al, Nature Reviews Neuroscience 2004; 5, 521-531, discloses that normal brain neurons express MHC class 1 molecules. WO2003/051834 discloses the identification in the brain of immune-related receptors, including PIRB.

### SUMMARY OF THE INVENTION

The present invention concerns the expression and function of Paired immunoglobulin-like receptor-B (PirB) in nervous tissue. PirB expressed in neurons acts to restrict regeneration and plasticity of neural circuits, and/or, restrict competition between circuits with different activity patterns or levels, for cortical representation. In the immune system, PirB functions through Shp-1 and Shp-2 phosphatases to inhibit signals that could lead to inappropriate and dangerous activation of immune cells toward normal, healthy cells. PirB regulates cytoskeletal dynamics, cell motility and adhesion, acting downstream of Src family kinases to modulate integrin activity. In neurons, PirB may have analogous activities in restricting the response of neurons to activity, only allowing limited plasticity. In the absence of functional PirB, rules regarding strengthening of synapses, or the formation of new synapses, or even outgrowth of new neuritis, may be altered to allow for exuberant and abnormal strengthening of those circuits with an activity-dependent advantage in competition for synaptic territory.

The invention is useful in methods of increasing nervous system plasticity in a subject comprising administering to the subject an agent which modulates the activity of Paired Immunoglobulin-like Receptor-B (PirB) in neurons of the subject. In more preferred embodiments, the agent acts as a receptor antagonist to PirB, wherein it can be a small molecule or an inhibitory peptide mimetic.

The invention is useful in methods of increasing nervous system plasticity in a subject comprising administering to the subject an agent which modulates the activity of, or signal transduction of PirB in neurons of the subject. Again, preferred embodiments include agents that act to decrease the activity or signal transduction of PirB and can be small molecules.

The invention is useful in methods of increasing nervous system plasticity in a subject comprising administering to the subject an agent which decreases the expression of PirB in neurons of the subject, particularly wherein the agent consists of one or more small interfering ribonucleic acids (siRNA).

The invention is useful in methods of treating various disorders and/or diseases associated with synaptic plasticity in a subject wherein the methods comprise the administration of an agent which decreases the activity, signal transduction or expression of PirB in neurons of the subject. These disorders and diseases include but are not restricted to CNS diseases, memory loss or lack of formation, such as Alzheimer's disease; learning disorders; developmental disabilities, such as autism, dyslexia or cerebral palsy; spinal cord injury; traumatic brain injury; stroke; and disorders or diseases involving the visual system.

The invention is useful in methods which in general comprise administering a therapeutically effective amount of one or more of the above described compounds (including but not limited to; small molecule, antibody, peptide mimetic, peptide fragment, siRNA) to a subject (e.g., a mammal, particularly human), that is suffering from or susceptible to a CNS disease.

The invention provides in a first aspect, an agent for use in treating a CNS disease or disorder in a subject, by increasing nervous system plasticity in the subject, which agent decreases the activity, signal transduction or expression of PirB in neurons of the subject, wherein the agent is selected from the group consisting of a polypeptide comprising the extracellular domain of PirB, an antibody directed to PirB, and an siRNA, ribozyme or antisense molecule capable of interfering with PirB expression.

In preferred embodiments, the invention provides such an agent for use in treating a CNS disease or disorder in a subject selected from the group consisting of autism, dyslexia, cerebral palsy, traumatic brain injury, spinal cord injury, stroke, Alzheimer's disease, and memory disorders, by increasing nervous system plasticity in a subject, which agent modulates the activity of PirB in neurons of the subject, wherein the agent is selected from the group consisting of a polypeptide comprising the extracellular domain of PirB, and an antibody directed to PirB.

In preferred embodiments, the invention provides such an agent for use in treating a CNS disease or disorder in a subject selected from the group consisting of autism, dyslexia, cerebral palsy, traumatic brain injury, spinal cord injury, stroke, Alzheimer's disease, and memory disorders, by increasing nervous system plasticity in a subject which agent modulates the signal transduction of PirB in neurons of the subject, wherein the agent is selected from the group consisting of a polypeptide comprising the extracellular domain of PirB, and an antibody directed to PirB.

In preferred embodiments, the invention provides such an agent for use in treating a CNS disease or disorder in a subject selected from the group consisting of autism, dyslexia, cerebral palsy, traumatic brain injury, spinal cord injury, stroke, Alzheimer's disease, and memory disorders, by increasing nervous system plasticity in a subject, which agent decreases the expression of PirB in neurons of the subject wherein the agent is an one or more siRNA's capable of interfering with PirB expression.

The CNS disease or disorder in a subject may be autism, dyslexia, cerebral palsy, traumatic brain injury, spinal cord injury, stroke, Alzheimer's disease, or memory disorders.

In an alternative aspect, the invention provides an agent for treating a visual system disorder or disease in a subject, by increasing nervous system plasticity in the subject, which decreases the activity, signal transduction or expression of PirB in neurons of the subject, wherein the agent is selected from the group consisting of a polypeptide comprising the extracellular domain of PirB, an antibody directed to PirB, and an siRNA, ribozyme or antisense molecule capable of interfering with PirB expression.

The invention also includes pharmaceutical compositions that comprise one or more of the above described compounds optionally mixed with a pharmaceutically acceptable carrier and optionally packaged together with instructions (e.g. written) for use of the composition for a condition as disclosed herein.

In addition, the invention is useful in methods for identifying and designing drugs which inhibit, regulate or decrease the activity, signal transduction and/or expression of PirB. This is useful in determining the therapeutic value of drugs and/or identification of novel candidate drug for neuronal treatment. For example: drugs for treating neurological diseases and disorders such as all agonists and antagonists that are known or designed to interact with PirB present in neurons or the downstream signaling pathways.

Furthermore, the invention is useful in a method of increasing levels ofPir-A in a subject comprising administering to the subject an agent which modulates the activity of PirB wherein the agent is selected from the group consisting of an antagonist to the PirB receptor, a small molecule antagonist of PirB, a peptide mimetic antagonist of PirB, an antibody directed to a PirB receptor and an antibody directed to a PirB specific ligand and a siRNA molecule capable of interfereing with PirB expression.

Other and further aspects, features and advantages of the present teachings will be apparent from the following description of the various embodiments of the present teachings given for the purpose of disclosure.

### BRIEF DESCRIPTION OF THE FIGURES

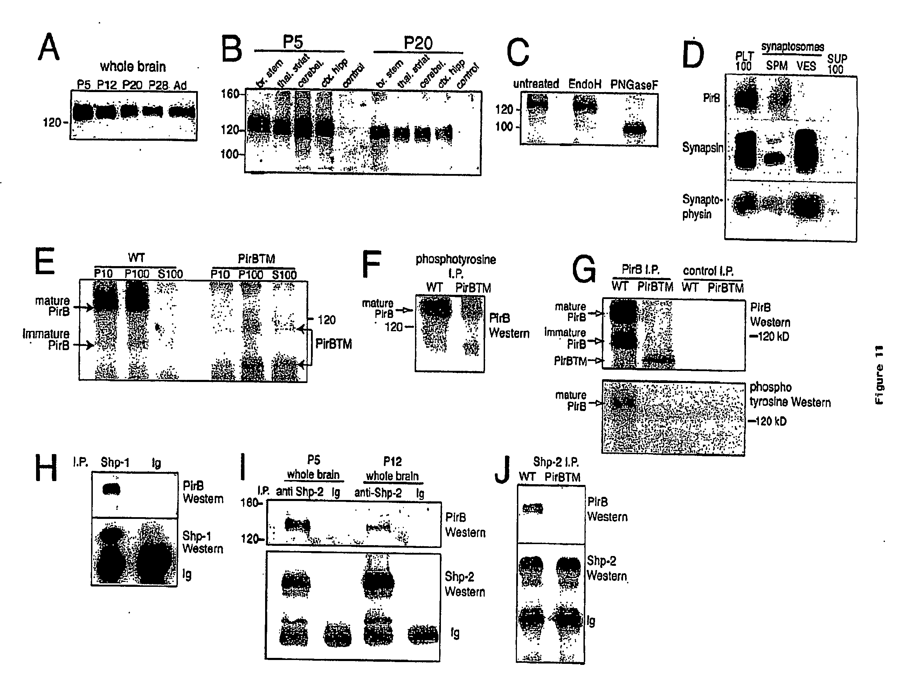

Figure 1. PirB mRNA is expressed in the mouse brain. 35S-labeled PirB-specific antisense or sense control probes representing the 3' region of PirB mRNA were used to detect PirB mRNA in sections from mouse brains of various ages. A. PO corona) section. B. P7 sagittal section. C. P14 sagittal section. D. P29 sagittal section. E. P25 coronal section. F. Adult sagittal section. Scale bars = 1 mm. G. Corona) section of a P9 brain immunostained with the anti-PirB antibody C19.

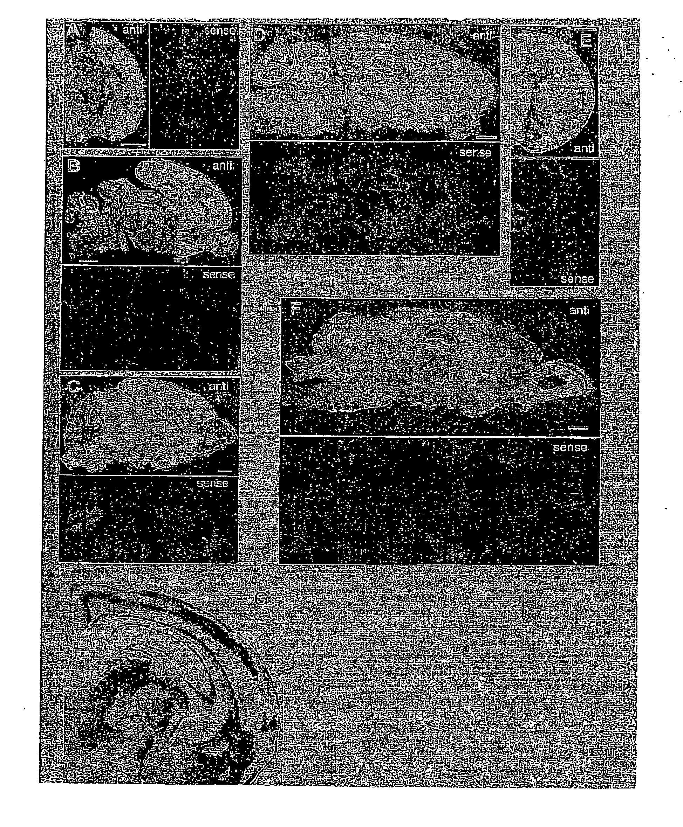

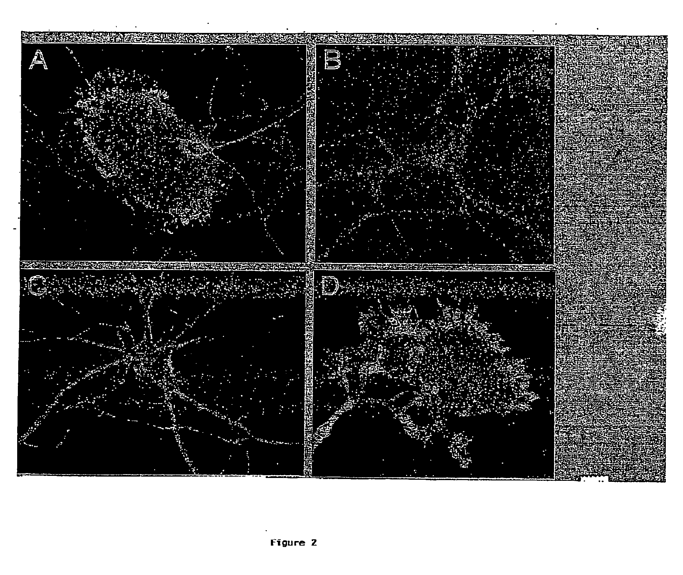

Figure 2. PirB immunoreactivity on cultured cortical neurons.A. Growth cone from a cortical neuron cultured three days and immunostained with anti-PirB antibody 1477 (red), actin binding protein phalloidin (blue) and synapsin (green). B. Cultured cortical neuron nine days in vitro immunostained with anti PirB antibody C 19, anti-PSD-95 (green) and DAPI (blue). C. Cultured cortical neuron nine days in vitro immunostained with anti PirB antibody A20 (red), and anti-neurofilament (green). D. Growth cone from a cortical neuron cultured three days and immunostained with anti-PirB antibody C19 (red), axonal growth cone marker GAP-43 (blue), and actin stain phalloidin (green).

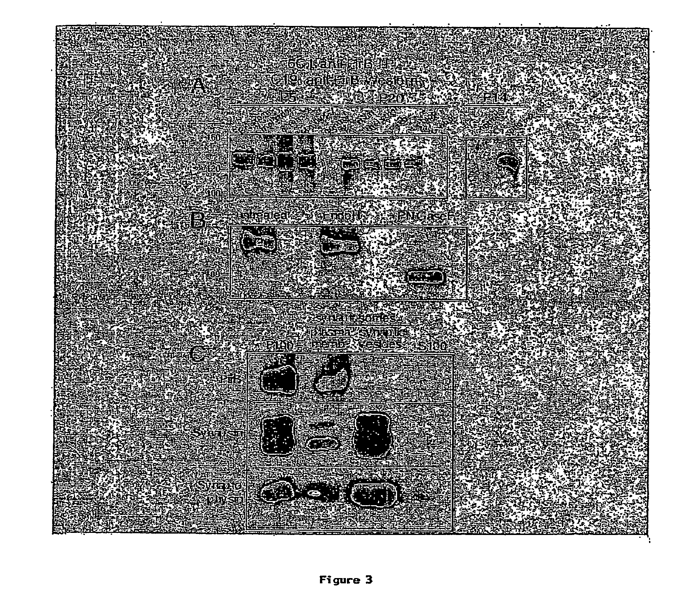

Figure 3. PirB protein from the mouse balb. A. PirB was immunoprecipitated from brain stem, thalamus and striatum, cerebellum, and cortex, hippocampus or optic nerve derived from equal total amounts of protein from P5 and P20 mouse brains (P14 for optic nerve) using the 6C 1 monoclonal antibody and detected by Western Blot with the polyclonal C19 antibody. Control I.P. was total rat ig G.I.P. from cerebellum lysate. B. PirB was immunoprecipitated from total mouse brain at P7 and treated with EndoH and PNGaseF glycosidases. C. Synaptosomal fractions were prepared (see methods), and PirB was immunoprecipitated from fractions. P100 = light membranes. synaptosomes were subfractionated into synaptic plasma membranes and synaptic v esicles by hypotontic shock S100 rep resents so kb le fraction. Synapsin and Synaptophysin, synaptic ve side proteins, were used as fractionation controls.

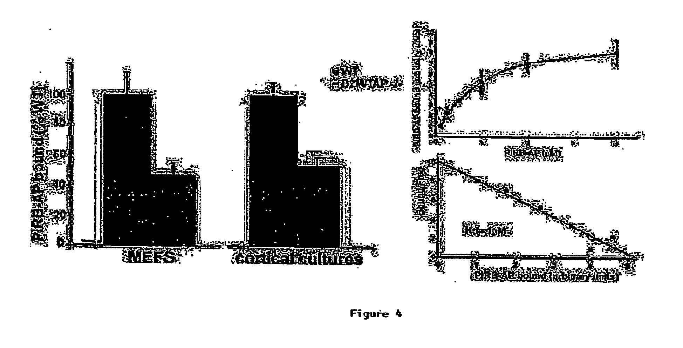

Figure 4. Solute PirBAP fusion protein binds to cultured cortical neurons in an MHC [dependent manner. PirBAP binds to WT mouse embyro fibroblasts (MEFs), and to culture cortical neurons. This binding is dramatically reduced in B2M/Tap mutant mice, where cell surface expression of MI-IC I molecules are abrogated (left). Scatchard p lot analysis reveal saturable binding awe, and a dissociation constant of approximately 1 *µ*M.

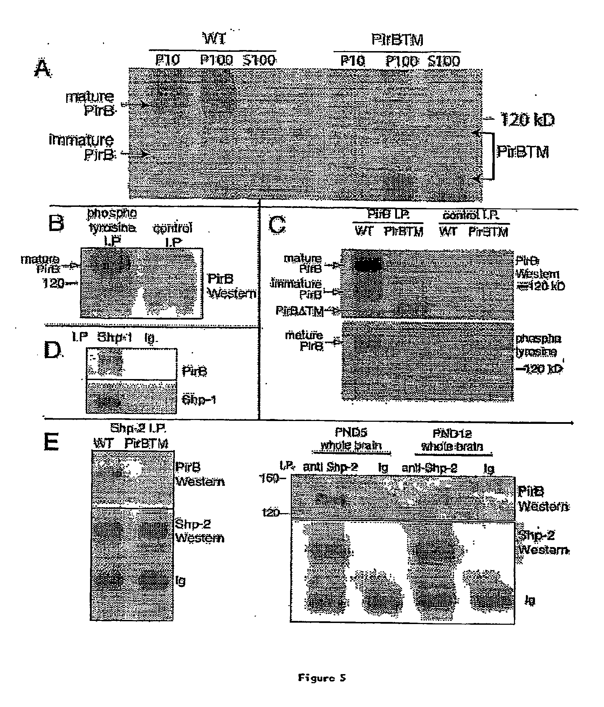

Figure 5. PirB function is abrogated in PirBTM mice. (A) Subcellular fractionation from wild-type and PirBTM mouse brains. In WT mice, 130 kD mature PirB fractionates with heavy membranes (P10) and light membranes (P100), and none is detected in soluble 5100 fraction. PirBTM is smaller, and exhibits increased solubility, as a large proportion appears in the soluble (S100) fraction. (B) Anti-phosphotyrosine immunoprecipitation followed by anti-PirB Western blot reveals that PirB is phosphorylated. (C) Anti-PirB I.P. followed by anti-PirB Western blot, which is then stripped and probed for anti-phosphotyrosine. Only WT PirB is phosphphorylated; no phosphorylated PirBTM is detected. (D) Anti-Shp-1 immunoprecipitation, followed by Shp-1 and PirB Western blots indicate a detectable interaction between Shp-1 and PirB in mouse brain. E.Anti-Shp-2 immunoprecipiation demonstrates a robust PirB/Shp-2 complex in the WT mouse brain, and no interaction in the PirBTM brain (left panel). Shp-2 immunoprecipitation from PND5 and PND20 brains also show this interaction. Controls were performed using non-specific Ig control immunoprecipitation from WT brain to demonstrate specificity of the Shp-2 antibody (right panel).

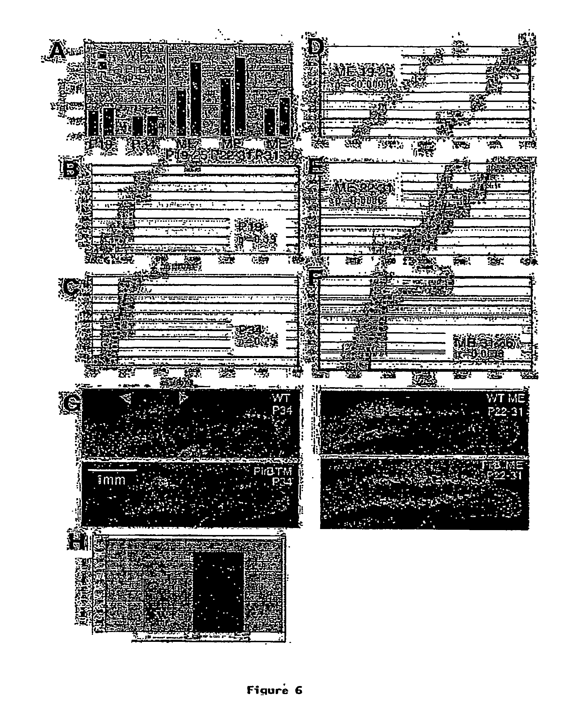

Figure 6. PirB restricts ocular dominance plasticity in the mouse visualcortex. (A) Average widths of ipsilateral Arc induction zones in P19 end P34 WT end PirBTM mice (left panel), and widths after monocular enucleation (ME) from P19-25, P22-31, and P31-36. No difference is seen in normal development, but ME produces a greater shift toward the non-deprived eye in PirBTM mice than in WT mice n=6-9 mice per condition. (B·F) Cumulative histograms of individual measurements at each age end under each condition. (G) Examples of ipsilateral Arc patch (yellow arrows) at P34 after refinement is complete in WT (top left) and PirBTM (bottom left), and expended patches after P22-31 ME in the right panels. (H) Width of 3H-proline a labeled ipsilateral patch is wider in PirBTM then WT mice after ME from P25-P40.

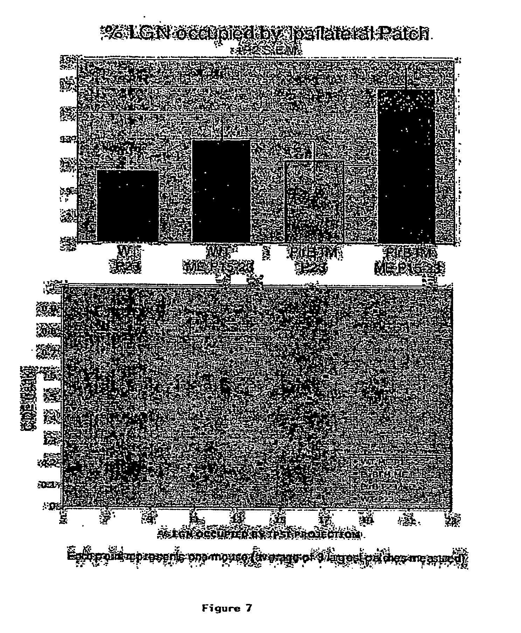

Figure 7. Plasticity in the segregated LG N of PirBTM mice. Anterograde track 1 g Indicates that the area of the LGN occupied by ipsiliateral axons is the same in WT and Pir3TM mice at P23. After ME from P15 to P23, the ipsilateral area in the PirBTM mice has expanded much more than WT.

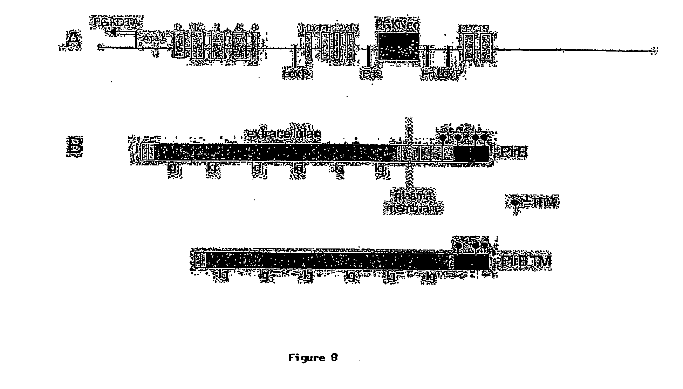

Figure 8. Schematic of targeting vector for PirBTM (see a methods). A. Light blue boxes indicate exons. Exons 10,11, 12, and 13, encoding PirB transmembrane domain end part of its intracellular domain are deleted upon exposure to Cre recombines. B. Schematic of hypothetical PirB and PirB TM mutant proteins. Ig=Ig domain, ITIM= Immunoreceptortyrosine-based inhibitorymotifs.

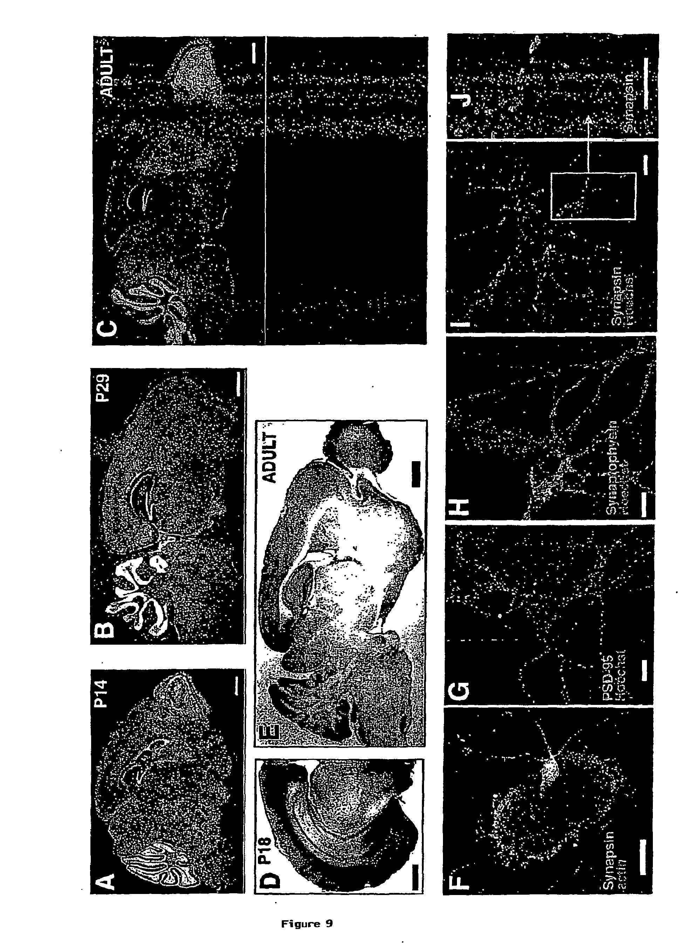

Figure 9 illustrates that PirB mRNA and protein are expressed in neurons wherein:
   A-C: 35-S labeled PirB-specific probes representing the 3' region of PirB mRNA were used to detect PirB mRNA in sections from mouse brains of various ages. In situ hybridizations are shown in darkfield optics (silver grains appear white);
      A. P 14 sagittal section;
      B. P29 sagittal section;
      C. Adult sagittal section (top) plus sense control (bottom);
   D-J: Immunohistochemistry using PirB-specific antibodies
      D. P 18 coronal section;
      E, adult sagittal section stained with anti-PirB antibody A20. Scale bars A-E = 1mm;
      F. Growth cone of a cortical neuron 3 DIV immunostained with anti-PirB 1477 (red) phalloidin (blue) and anti-synapsin (green);
      G. Cortical neuron 18 DIV stained with anti-PirB (red), postsynaptic marker PSD-95 (green), and Hoechst (blue);
      H. Cortical neuron 14 DIV stained with anti-PirB (red), presynaptic protein synaptophysin (green), and Hoechst (blue);
      I. Cortical neuron 14 DIV stained with anti-PirB (red), presynaptic protein synapsin (green), and Hoechst (blue); and
      J. Higher magnification of I. Scale bars F-J=10um.

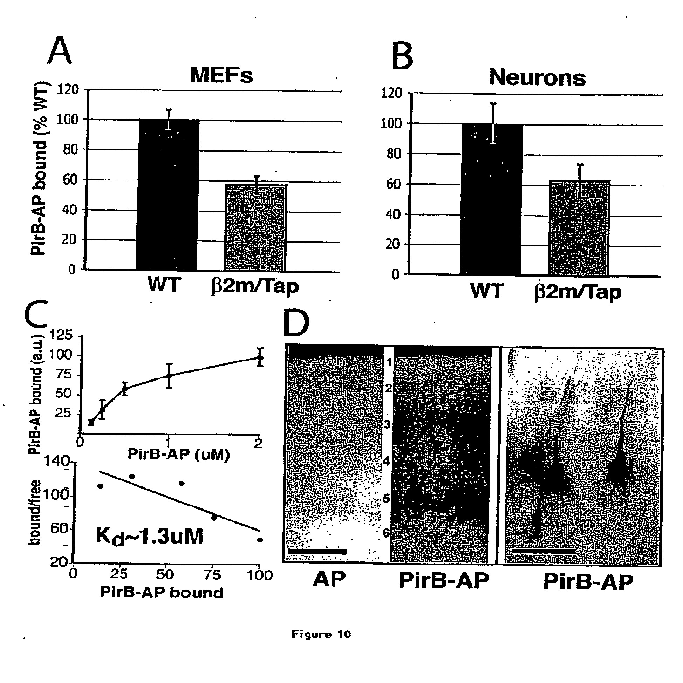

Figure 10 illustrates that soluble PirB binds to neurons wherein;
   Soluble PirB-AP binds to mouse embryo fibroblasts (MEFs) (A), and to cultured cortical neurons (B); binding is dependent on surface expression of MHCI protein, as binding is reduced significantly in cultures derived from ß2m-/-/Tap1-/- mice (p<0.01 for MEFs and p= 0.03 for neurons); (C) Binding of PirB-AP to neurons is saturable (top); Scatchard analysis (bottom) predicts a dissociation constant of ~ 1.3 µM. (D) PirB-AP binds to pyramidal neurons in sections of cortex. Numbers indicate cortical layers. Scale bars = 250 um (left, middle panels) or 50 um (right panel).
Figure 11 illustrates that PirB protein is expressed thoughout the brain and forms complexes with Shp-1 and Shp-2;
   A. PirB was immunoprecipitated using 6C 1 monoclonal antibody and detected by Western blot using C19 polyclonal antibody, from whole brain at postnatal day 5, P12, P20, P28, or adult (Ad);
   B. PirB protein immunoprecipitated from brain stem, thalamus and striatum (thal. striat), cerebellum, and cortex/hippocampus (ctx. hipp) derived from equal amounts of protein from P5 or P20 mouse brains. Control I.P. was non-specific rat IgG in lysate from cerebellum;
   C. PirB was immunoprecipitated from whole brain at P7 and treated with EndoH or PNGaseF glycosidases;
   D. PirB was immunoprecipitated from synaptosomal fractions (see methods). PLT100 = light membranes, SPM = synaptic plasma membranes, VES = synaptic vesicle fraction, SUP100 = soluble fraction. Synapsin and Synaptophysin synaptic vesicle proteins were used as fractionation markers;
   E. Subcellular fractionation from WT and PirBTM mouse brains. In WT, 130 kD mature PirB fractionates with heavy membranes (P10 fraction) and light membranes (P100 fraction), but none is detected in soluble S100 fraction. In contrast, mutant PirBTM is smaller and exhibits increased solubility, as a large proportion appears in the soluble (S 100) fraction;
   F. Anti-phosphotyrosine immunoprecipitation followed by anti-PirB Western blot reveals that PirB is phosphorylated, and no signal is detected in PirBTM mice;
   G. Anti-PirB I.P. followed by anti-PirB Western blot, which is then stripped and probed for anti-phosphotyrosine. Only WT PirB is phosphorylated; no phosphorylated PirBTM is detected;
   H. Anti-Shp-1 I.P., followed by Shp-1 and PirB Western blots demonstrates that PirB interacts with Shp-1 in brain;
   I. Anti-Shp-2 I.P. followed by Shp-2 and PirB Western blots from P5 or P12 brains demonstrate PirB/Shp-2 complex in the brain; and
   J. PirBTM/Shp-2 complex is absent in PirBTM brains.

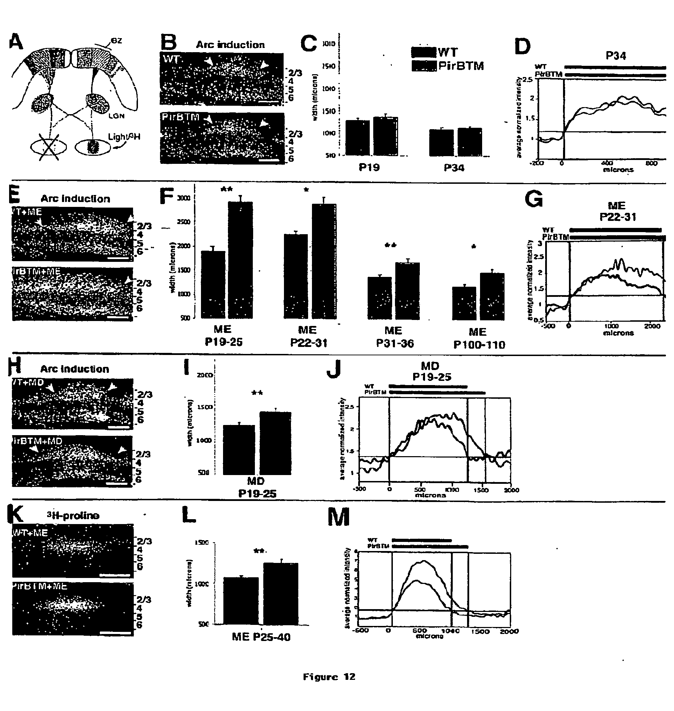

Figure 12 illustrates enhanced OD plasticity in visual cortex of PirBTM mice; Arc mRNA induction (A-G), transneuronal autoradiography (H-J);
   A. Schematic of visual system showing connections from retina to lateral geniculate nucleus (LGN) to visual cortex. Note small binocular zone (BZ) that receives visual inputs via the LGN from both eyes;
   B-D: Developmental restriction of ipsilateral eye representation proceeds normally in PirBTM mice;
      B. Arc mRNA induced by visual stimulation of P34 mice, detected by in situ hybridization in cortex ipsilateral to stimulated eye. Yellow arrowheads delineate zone of Arc induction, which corresponds to the binocular zone. In normally reared mice at P19 or at P34, the width of Arc induction in WT or in PirBTM mice appears indistinguishable;
      C. Histograms represent average widths of Arc induction in layer 4. P19: WT n= 9, PirBTM n=7; P34: WT n= 7, PirBTM n=7, 3-4 sections/animal;
      D. Averaged line scans from all WT (blue) or PirBTM (red) sections at P34. Scans were made blind to genotype and were aligned at left border of BZ (black vertical line, left) Blue or red vertical line indicates right border of Arc induction;
   E-G: OD plasticity is enhanced in PirBTM mice;
      E. OD plasticity following monocular enucleation (ME) from P22-31 as assessed by Arc induction. Note expansion in width of in situ hybridization pattern after ME in WT (compare E top with B top). Zone of Arc induction after ME is even more extensive in PirBTM mice (E bottom panel) than WT (top panel);
      F. Consistently enhanced expansion in width of Arc induction in PirBTM vs WT mice following periods of ME from P19-25, P22-31, P31-36, or P100-110. Histograms represent average width of Arc induction. ME from P 19-25: WT n= 5, PirBTM n=9; P21-32: WT n= 6, PirBTM n=6; P31-36 WT n= 9, PirBTM n=9; P11-110: WT n= 5, PirBTM n=5, 3-4 sections/animal;
      G. Averaged line scans of layer 4 Arc signal in all WT (blue) or PirBTM (red) sections following ME from P22-31. Scans aligned at left border of BZ (vertical line, left). Blue or red vertical lines indicate right border. Note larger width in PirBTM mice;
   H-J: Transneuronal autoradiography reveals an increase in width of anatomical connections between LGN neurons representing ipsilateral eye and layer 4 of cortex following ME (P25-40) in PirBTM mice;
      H. Darkfield autoradiographs showing increased width of transneuronally transported radioactive label representing input from the ipsilateral eye in layer 4 of cortex in PirBTM (bottom) vs WT (top) mice;
      I. Histograms are averages from all mice (WT n=7, PirBTM n=6); and
      J. Averaged line scans from all WT or PirBTM sections. Scans were aligned at left border ofBZ (black vertical line, left). Blue or red vertical lines indicate width of thalamocortical projection in WT or PirBTM. Error bars in C,F,I = 1 S.E.M. asterisks: * = p<0.05; **=p<0.01.

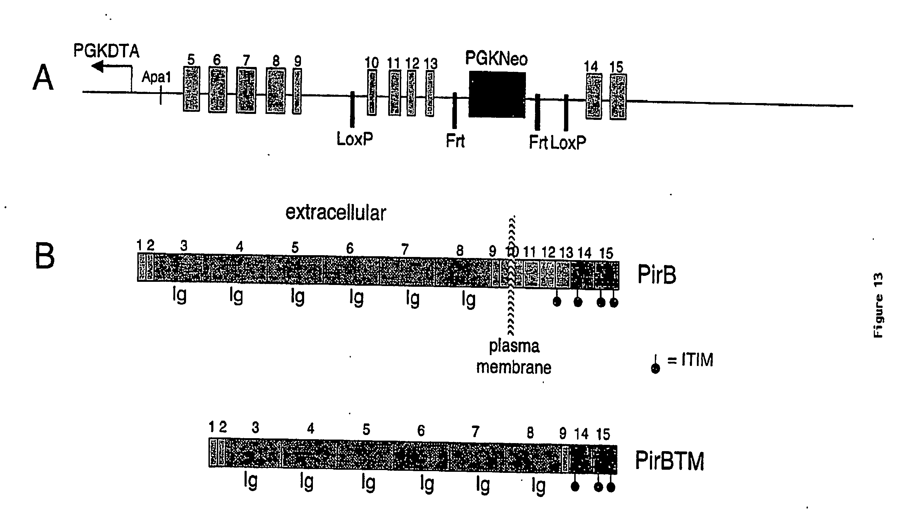

Figure 13 illustrates:
   A. Schematic of targeting vector for PirBTM (see methods). Light blue boxes indicate exons. Exons 10,11,12, and 13, encoding PirB transmembrane domain and part of the intracellular domain are deleted upon exposure to Cre recombinase; and
   B. Schematic of hypothetical PirB and PirBTM mutant proteins. Ig=Ig domain, ITIM = Immunoreceptor tyrosine-based inhibitory motifs. Colored boxes indicate protein domain encoded by separate exons. Exon indicated by numbers above box.

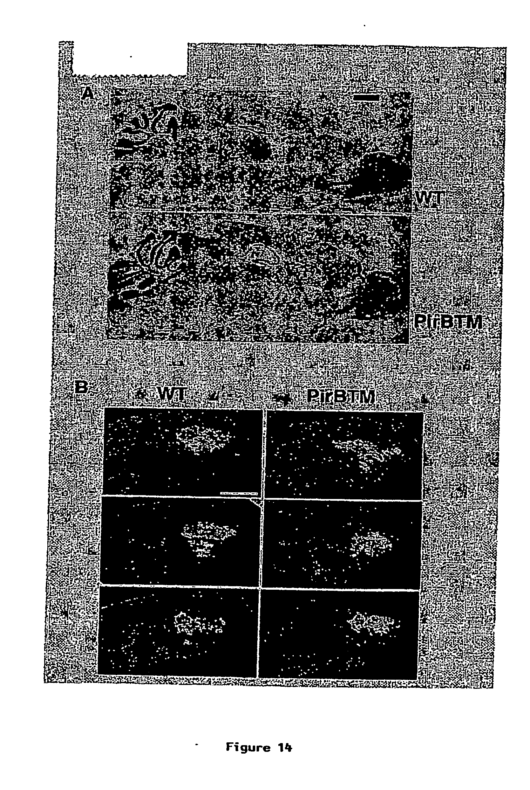

Figure 14 illustrates:
   A. Sagittal sections (20µm) of P30 WT (top) and PirBTM (bottom) mice stained with cresyl violet. Gross histological organization of the brain is indistinguishable from normal in PirBTM mice; Scale bar= 1 mm; and
   B. Anterograde tracing reveals normal patterns of eye-specific segregation of retinogeniculate axons in LGN of PirBTM mice. Pattern of retinogeniculate axons in three WT mice (left) and three PirBTM mice (right) appear similar, Scale bar= 100 um.

### DETAILED DESCRIPTION OF THE INVENTION

It has long been known that there are critical periods during brain development when experience can rapidly alter brain circuits by changing synaptic connections, both by altering the structure of connections and by changing their strength functionally (39). These critical periods are defined by the rapidity and ease with which neural connections can be changed by experience, such as monocular visual deprivation or eye removal, and such periods are thought to be terminated by progressive developmental changes leading to the adult state in which plasticity is far more limited if it occurs at all. It has generally been thought that the critical period for the effects of MD in visual cortex comes to a close due to the down regulation or removal of factors that enable early synaptic plasticity. However, the genetic loss of function experiments described here demonstrate a role for PirB in limiting the extent of synaptic plasticity present both in the thalamus and in the visual cortex, an effect that is likely to be involved in other regions of the central nervous system. In the absence of PirB function, plasticity in both of these structures remains immature in the sense that it is rapid and extensive even at older ages. This discovery is exciting because it implies that there are not only positive, but also negative regulators of synaptic plasticity and that by removing or reducing activity or levels of a negative regulator- PirB- it is possible to restore early, more robust forms of plasticity that may have been present all along even in the adult brain.

The implications of this discovery are broad. PirB is expressed throughout the brain and spinal cord, and thus may play a role in restricting plasticity in many neural systems beyond the visual system. For example, PirB is expressed in the hippocampus and cerebral cortex, structures known to function in learning and memory formation and consolidation by means of synaptic plasticity and circuit refinement. Thus, PirB, and its human homologs the LILRB/LIR/ILT family of proteins, represent prime candidate targets for therapeutics for alleviating the memory loss due to aging and Alzheimer's disease, and in cases for treatment of developmental disability such as autism, dyslexia or cerebral palsy. ,

In the immune system, PirB regulates cytoskeletal dynamics by inhibiting integrin signaling and chemokine signaling. Neuronal PirB may function in a similar way, restricting synaptic plasticity by limiting neuronal cytoskeletal activity, and thereby limiting neurite outgrowth and synapse remodeling. Release of these limitations through targeted inhibition of PirB or its downstream effector molecules may significantly enhance regeneration of neural circuits that have been lost due to spinal cord injury, head injury or stroke. In addition, recovery from stroke may be enhanced by coupling rehabilitation and retraining therapies with targeted inhibition of PirB pharmacologically, with the idea that the retraining of remaining, undamaged brain circuits can be facilitated by allowing them access to earlier, more robust forms of synaptic plasticity.

Conversely, the epileptic brain suffers from inappropriate activity-induced neuronal sprouting and outgrowth. Epilepsy runs in families, and thus has a genetic component. People suffering from epilepsy may have reduced PirB/LILRB activity due to genetic factors, or may benefit from limiting the extent of seizure-induced plasticity by enhancing PirB function through therapeutic intervention. The fundamental concept here is that a substrate for the more robust forms of synaptic plasticity characteristic of the developing brain are still present in the adult brain, but that intact PirB functions in adulthood to put a brake on the extent of neural plasticity. Thus, inhibitors of neural plasticity identified and designed as function-blocking therapeutics for recovery from injury and stroke, for enhancement of learning and memory, and as function-enhancing therapeutics for preventing unwanted changes in neural circuits that can occur with epilepsy or other forms of abnormal brain activity.

Here we identify the Paired pmmunoglobulin-like receptor-B, PirB, as a candidate neuronal receptor for MHCI. PirB is a transmembrane MHCI receptor expressed on various types of leukocytes (*9-15*), and has been shown to bind a broad array of MHCI molecules, as well as to bind directly to the requisite MHCI co-subunit β2microglobulin (*12, 13*, *16*). Our work demonstrates that PirB is expressed in subsets of neurons throughout the mouse brain at all ages tested. Cultured cortical and hippocampal neurons exhibit PirB immunostaining along neuronal axons and growth cones. Neuronal PirB protein is glycosylated, phosphorylated, and fractionates in part with synaptosomes. As in the immune system (*17*-*21*) neuronal PirB forms complexes with the phosphatases Shp-1 and Shp-2. Functional experiments in mutant mice reveal that ocular dominance plasticity in the visual cortex is more robust, and that segregated retinal ganglion axon are unusually plastic in the absence of PirB signaling. These results demonstrate that a neuronal-expressed MHCI receptor acts to limit visual system plasticity.

### Definitions

It is understood that this invention is not limited to the particular materials and methods described herein. It is also to be understood that the terminology used herein is for the purpose of describing particular embodiments and is not intended to limit the scope of the present invention which will be limited only by the appended claims. As used herein, the singular forms "a", "an", and "the" include plural reference unless the context clearly dictates otherwise. For example, a reference to "leukocytes" includes a plurality of cells known to those skilled in the art.
Unless defined otherwise, all technical and scientific terms used herein have the same meanings as commonly understood by one of ordinary skill in the art to which this invention belongs. All publications mentioned herein are cited for the purpose of describing and disclosing the models, protocols and reagents which are reported in the publications and which might be used in connection with the invention. Nothing herein is to be construed as an admission that the invention is not entitled to antedate such disclosure by virtue of prior invention.

As used herein, a "small molecule" is usually less than about 25K in molecular weight, preferably less than 10K and may possess a number of physicochemical and pharmacological properties which enhance cell penetration, allow it to resist degradation and prolong its physiological half-life. Preferably, small molecules are not immunogenic. In addition, the small molecule should be amenable to high throughput screening.

As used herein, "Expression" refers to the transcription and/or translation of an endogenous gene (PirB), heterologous gene or nucleic acid segment, or a transgene in cells. For example, in the case of siRNA constructs, expression may refer to the transcription of the siRNA only. In addition, expression refers to the transcription and stable accumulation of sense (mRNA) or functional RNA. Expression may also refer to the production of protein.

As used herein, the term "administering a molecule to a cell" (e.g., an expression vector, nucleic acid, cytokines, angiogenic factors, a delivery vehicle, agent, and the like) refers to transducing, transfecting, microinjecting, electroporating, or shooting, the cell with the molecule. In some aspects, molecules are introduced into a target cell by contacting the target cell with a delivery cell (e.g., by cell fusion or by lysing the delivery cell when it is in proximity to the target cell).

As used herein, "MHC Class I molecules" refers to classical (class 1a) MHC I molecules (HLA-A, -B, -C, -G and the like) and other non-classical (class 1b) MHC Class I molecules. MHC Class I molecules include human MHC Class I molecules (the human leukocyte antigen (HLA) complex) and vertebrate equivalents thereof, such is Class I antigens of the H-2 locus of mice, in particular H-2 D and K. Human MHC Class I antigens include, for example, HLA-A, B, C, Qa and T1. In addition, HLA-G encodes nonclassical MHC class I products. There are also numerous MHC class I-like genes, many of which are coded outside of the canonical MHC Class I region, including HFE, MICA, MICB, CD1-a, -b, -c, -d, and members of the ULPB family. Source for both human and mouse data:
http://imgt.cines.fr/textes/IMGTrepertoireMHC/LocusGenes/nomenclatures/mous e/MHC/Mu_MHCnom.html

The PirB receptor has been identified recently in mice on the basis of their homology with the human Fcα receptor (FcαR). PirB shares sequence similarity with a gene family that includes human FcαR and killer inhibitory receptors (KIR), mouse gp49, bovine Fc receptor for IgG (FcγR), and the recently identified human Ig-like transcripts (ILT)/leukocyte Ig-like receptors (LIR)/monocyte/macrophage Ig-like receptors (MIR). The PirB gene is located on mouse chromosome 7 in a region syntenic with the human chromosome 19q13 region that contains the *Fc*α*R*, *KIR,* and *ILT*/*LIR*/*MIR* genes. DNA sequences for PirB predict type I transmembrane proteins with similar ectodomains (>92% homology) each containing six Ig-like domains. The PirB protein, encoded by the Pirb gene, has a typical uncharged transmembrane region and a long cytoplasmic tail with multiple candidate immunoreceptor tyrosine-based inhibitory motifs (ITIMs). Recent studies have demonstrated the inhibitory function of the two most membrane-distal ITIM units in the PirB cytoplasmic region. The PirB inhibitory function is mediated through ITIM recruitment of the protein tyrosine phosphatase SHP-1.

It should be appreciated that the above PirB not only refers to the polypeptide, but also the gene and all currently known variants thereof, including the different mRNA transcripts to which the gene and its variants can give rise, and any further gene variants which may be elucidated. In general, however, such variants will have significant homology (sequence identity) to a sequence of a table above, e.g. a variant will have at least about 70 percent homology (sequence identity) to a sequence of the above tables 1-5, more typically at least about 75, 80, 85, 90, 95, 97, 98 or 99 homology (sequence identity) to a sequence of the above tables 1-5. Homology of a variant can be determined by any of a number of standard techniques such as a BLAST program.

"Central nervous system diseases or disorders as used herein refers to any neurological disorder whose disease course or severity could be either prevented or treated by the teachings of the current invention; that is, could be benefited by a therapeutic enhancing neuronal plasticity or regeneration; including but not limited to neurodegenerative disorders (Parkinson's; Alzheimer's) or autoimmune disorders (multiple sclerosis) of the central nervous system; memory loss; long term and short term memory disorders; learning disorders; autism, depression, benign forgetfulness, childhood learning disorders, close head injury, and attention deficit disorder; autoimmune disorders of the brain, neuronal reaction to viral infection; brain damage; depression; psychiatric disorders such as bi-polarism, schizophrenia and the like; narcolepsy/sleep disorders(including circadian rhythm disorders, insomnia and narcolepsy); severance of nerves or nerve damage; severance of the cerebrospinal nerve cord (CNS) and any damage to brain or nerve cells; neurological deficits associated with AIDS; tics (e.g. Giles de la Tourette's syndrome); Huntington's chorea, schizophrenia, traumatic brain injury, tinnitus, neuralgia, especially trigeminal neuralgia, neuropathic pain, inappropriate neuronal activity resulting in neurodysthesias in diseases such as diabetes, MS and motor neurone disease, ataxias, muscular rigidity (spasticity) and temporomandibular joint dysfunction; Reward Deficiency Syndrome (RDS) behaviors in a subject.

"Diagnostic" means identifying the presence or nature of a pathologic condition. Diagnostic methods differ in their sensitivity and specificity. The "sensitivity" of a diagnostic assay is the percentage of diseased individuals who test positive (percent of "true positive"). Diseased individuals not detected by the assay are "false negatives." Subjects who are not diseased and who test negative in the assay, are termed "true negatives." The "specificity" of a diagnostic assay is 1 minus the false positive rate, where the "false positive" rate is defined as the proportion of those without the disease who test positive. While a particular diagnostic method may not provide a definitive diagnosis of a condition, it suffices if the method provides a positive indication that aids in diagnosis.

As used herein, a "pharmaceutically acceptable" component is one that is suitable for use with humans and/or animals without undue adverse side effects (such as toxicity, irritation, and allergic response) commensurate with a reasonable benefit/risk ratio.

The terms "patient" or "individual" are used interchangeably herein, and is meant a mammalian subject to be treated, with human patients being preferred. In some cases, the methods of the invention find use in experimental animals, in veterinary application, and in the development of animal models for disease, including, but not limited to, rodents including mice, rats, and hamsters; and primates.

As used herein, "treatment" refers to a symptom which approaches a normalized value, e.g., is less than 50% different from a normalized value, preferably is less than about 25% different from a normalized value, more preferably, is less than 10% different from a normalized value, and still more preferably, is not significantly different from a normalized value as determined using routine statistical tests. For example, treatment of depression includes, for example, relief from the symptoms of depression which include, but are not limited to changes in mood, feelings of intense sadness and despair, mental slowing, loss of concentration, pessimistic worry, agitation, and self-deprecation. Physical changes may also be relieved, including insomnia, anorexia and weight loss, decreased energy and libido, and the return of normal hormonal circadian rhythms. Another example, when using the terms "treating Parkinson's disease" or "ameliorating" as used herein means relief from the symptoms of Parkinson's disease which include, but are not limited to tremor, bradykinesia, rigidity, and a disturbance of posture.

"Cells of the immune system" or "immune cells" as used herein, is meant to include any cells of the immune system that may be assayed, including, but not limited to, B lymphocytes, also called B cells, T lymphocytes, also called T cells, natural killer (NK) cells, lymphokine-activated killer (LAK) cells, monocytes, macrophages, neutrophils, granulocytes, mast cells, platelets, Langerhans cells, stem cells, dendritic cells, peripheral blood mononuclear cells, tumor-infiltrating (TIL) cells, gene modified immune cells including hybridomas, drug modified immune cells, and derivatives, precursors or progenitors of the above cell types.

"Immune effector cells" refers to cells capable of binding an antigen and which mediate an immune response. These cells include, but are not limited to, T cells (T lymphocytes), B cells (B lymphocytes), monocytes, macrophages, natural killer (NK) cells and cytotoxic T lymphocytes (CTLs), for example CTL lines, CTL clones, and CTLs from tumor, inflammatory, or other infiltrates.

"T cells" or "T lymphocytes" are a subset of lymphocytes originating in the thymus and having heterodimeric receptors associated with proteins of the CD3 complex (e.g., a rearranged T cell receptor, the heterodimeric protein on the T cell surfaces responsible for antigen/MHC specificity of the cells). T cell responses may be detected by assays for their effects on other cells (e.g., target cell killing, macrophage, activation, B-cell activation) or for the cytokines they produce.

"Activity", "activation" or "augmentation" is the ability of "resting" immune cells to respond and exhibit, on a measurable level, an immune function. Measuring the degree of activation refers to a quantitative assessment of the capacity of immune cells to express enhanced activity when further stimulated as a result of prior activation. The enhanced capacity may result from biochemical changes occurring during the activation process that allow the immune cells to be stimulated to activity in response to low doses of stimulants.

As used herein, the term "polypeptide" comprises amino acid chains of any length, including full length proteins comprising the sequences recited herein. A polypeptide comprising an epitope of a protein comprising a sequence as described herein may consist entirely of the epitope, or may contain additional sequences. The additional sequences may be derived from the native protein or may be heterologous, and such sequences may (but need not) possess immunogenic or antigenic properties.

The terms "specific binding" or "specifically binding", as used herein, in reference to the interaction of an antibody and a protein or peptide, mean that the interaction is dependent upon the presence of a particular structure (i.e., the antigenic determinant or epitope) on the protein; in other words, the antibody is recognizing and binding to a specific protein structure rather than to proteins in general. For example, if an antibody is specific for epitope "A", the presence of a protein comprising epitope A (or free, unlabeled A) in a reaction comprising labeled "A" and the antibody will reduce the amount of labeled A bound to the antibody. "Specific binding" in general, refers to any MHC Class I molecule binding to its ligand, such as for example the binding of a T cell receptor expressed by a T lymphocyte, to an MHC molecule and peptide on an antigen presenting cell.

As used herein, the term "antibody" refers to a polypeptide or group of polypeptides which are comprised of at least one binding domain, where an antibody binding domain is formed from the folding of variable domains of an antibody molecule to form three-dimensional binding spaces with an internal surface shape and charge distribution complementary to the features of an antigenic determinant of an antigen, which allows an immunological reaction with the antigen. Antibodies include recombinant proteins comprising the binding domains, as wells as fragments, including Fab, Fab', F(ab)₂, and F(ab')₂ fragments. The term "antibody," as used herein, also includes antibody fragments either produced by the modification of whole antibodies or those synthesized de novo using recombinant DNA methodologies. It also includes polyclonal antibodies, monoclonal antibodies, chimeric antibodies, humanized antibodies, or single chain antibodies. "Fc" portion of an antibody refers to that portion of an immunoglobulin heavy chain that comprises one or more heavy chain constant region domains, CH₁, CH₂ and CH₃, but does not include the heavy chain variable region.

An "epitope", as used herein, is a portion of a polypeptide that is recognized (i.e., specifically bound) by a B-cell and/or T-cell surface antigen receptor. Epitopes may generally be identified using well known techniques, such as those summarized in Paul, Fundamental Immunology, 3rd ed., 243-247 (Raven Press, 1993) and references cited therein. Such techniques include screening polypeptides derived from the native polypeptide for the ability to react with antigen-specific antisera and/or T-cell lines or clones. An epitope of a polypeptide is a portion that reacts with such antisera and/or T-cells at a level that is similar to the reactivity of the full length polypeptide (e.g., in an ELISA and/or T-cell reactivity assay). Such screens may generally be performed using methods well known to those of ordinary skill in the art, such as those described in Harlow and Lane, Antibodies: A Laboratory Manual, Cold Spring Harbor Laboratory, 1988. B-cell and T-cell epitopes may also be predicted via computer analysis. Polypeptides comprising an epitope of a polypeptide that is preferentially expressed in a tumor tissue (with or without additional amino acid sequence) are also disclosed.

The terms "nucleic acid molecule" or "polynucleotide" will be used interchangeably throughout the specification, unless otherwise specified. As used herein, "nucleic acid molecule" refers to the phosphate ester polymeric form of ribonucleosides (adenosine, guanosine, uridine or cytidine; "RNA molecules") or deoxyribonucleosides (deoxyadenosine, deoxyguanosine, deoxythymidine, or deoxycytidine; "DNA molecules"), or any phosphoester analogues thereof, such as phosphorothioates and thioesters, in either single stranded form, or a double-stranded helix. Double stranded DNA--DNA, DNA-RNA and RNA--RNA helices are possible. The term nucleic acid molecule, and in particular DNA or RNA molecule, refers only to the primary and secondary structure of the molecule, and does not limit it to any particular tertiary forms. Thus, this term includes double-stranded DNA found, *inter alia,* in linear or circular DNA molecules (e.g., restriction fragments), plasmids, and chromosomes. In discussing the structure of particular double-stranded DNA molecules, sequences may be described herein according to the normal convention of giving only the sequence in the 5' to 3' direction along the nontranscribed strand of DNA (i.e., the strand having a sequence homologous to the mRNA). A "recombinant DNA molecule" is a DNA molecule that has undergone a molecular biological manipulation.

As used herein, the term "fragment or segment", as applied to a nucleic acid sequence, gene or polypeptide, will ordinarily be at least about 5 contiguous nucleic acid bases (for nucleic acid sequence or gene) or amino acids (for polypeptides), typically at least about 10 contiguous nucleic acid bases or amino acids, more typically at least about 20 contiguous nucleic acid bases or amino acids, usually at least about 30 contiguous nucleic acid bases or amino acids, preferably at least about 40 contiguous nucleic acid bases or amino acids, more preferably at least about 50 contiguous nucleic acid bases or amino acids, and even more preferably at least about 60 to 80 or more contiguous nucleic acid bases or amino acids in length. "Overlapping fragments" as used herein, refer to contiguous nucleic acid or peptide fragments which begin at the amino terminal end of a nucleic acid or protein and end at the carboxy terminal end of the nucleic acid or protein. Each nucleic acid or peptide fragment has at least about one contiguous nucleic acid or amino acid position in common with the next nucleic acid or peptide fragment, more preferably at least about three contiguous nucleic acid bases or amino acid positions in common, most preferably at least about ten contiguous nucleic acid bases amino acid positions in common.

A significant "fragment" in a nucleic acid context is a contiguous segment of at least about 17 nucleotides, generally at least 20 nucleotides, more generally at least 23 nucleotides, ordinarily at least 26 nucleotides, more ordinarily at least 29 nucleotides, often at least 32 nucleotides, more often at least 35 nucleotides, typically at least 38 nucleotides, more typically at least 41 nucleotides, usually at least 44 nucleotides, more usually at least 47 nucleotides, preferably at least 50 nucleotides, more preferably at least 53 nucleotides, and in particularly preferred embodiments will be at least 56 or more nucleotides.

Further, an MHC Class I gene allelic variant may be isolated from, for example, human nucleic acid, by performing PCR using the pan specific probes as described in detail in the Examples section, e.g. Pan PIRB probe.. For example, the template for the reaction may be cDNA obtained by reverse transcription of mRNA prepared from, for example, human or non-human cell lines or tissue known or suspected to express a PIR gene or allelic variant thereof. Preferably, the allelic variant will be isolated from an individual who has a PIR mediated neuronal disorder. This method is also used to determine the absence of any MHC Class I expression.

In another aspect of the invention it is desirable to correct abnormal MHC Class I levels of expression or abnormal expression patterns. For example, MHC Class I and Class I-like gene sequences or portions thereof can be used in gene replacement therapy. Specifically, one or more copies of a normal class I MHC gene or a portion of the class I MHC gene that directs the production of a class I MHC gene product exhibiting normal class I MHC gene function, may be inserted into the appropriate cells within a patient, using vectors that include, but are not limited to adenovirus, adeno-associated virus, and retrovirus vectors, in addition to other particles that introduce DNA into cells, such as liposomes. Preferably the vector is herpes simplex virus vector, such as that described in US Patent No's: 5,501,979 and 5,661,033.

Additional methods that may be utilized to increase, decrease or modulate the overall level of PirB expression and/or PirB gene product activity include the introduction of appropriate PirB -expressing cells, preferably autologous cells, and/or stem cells, and/or neural progenitor cells into a patient at positions and in numbers that are sufficient to rectify the expression of PirB receptors.

Such cell-based gene therapy techniques are well known to those skilled in the art, see, e.g., Anderson, U.S. Pat. No. 5,399,349.

Additionally, compounds, such as those described, above and below, that are capable of modulating PirB activity can be administered using standard techniques that are well known to those of skill in the art. In instances in which the compounds to be administered are to involve an interaction with brain cells, the administration techniques should include well known ones that allow for a crossing of the blood-brain barrier.

Modulators of PirB include, but are not limited to: small molecules, antibodies, peptides, nucleic acids, protein or nucleic acid aptamers, antisense molecules, ribozymes, triple helix molecules, carbohydrates, and the like. Preferred modulators include those that up-regulate, such as for example, one involved in neural plasticity or in other cases a down regulator, such as for example a compound that inhibits (i.e., PirB) neuronal cell plasticity.

Libraries of compounds may be screened to identify modulators. There are a number of different libraries used for the identification of small molecule modulators, including: (1) chemical libraries, (2) natural product libraries, and (3) combinatorial libraries comprised of random peptides, oligonucleotides or organic molecules. Chemical libraries consist of random chemical structures, some of which are analogs of known compounds or analogs of compounds that have been identified as "hits" or "leads" in other drug discovery screens, some of which are derived from natural products, and some of which arise from non-directed synthetic organic chemistry. Natural product libraries are collections of microorganisms, animals, plants, or marine organisms which are used to create mixtures for screening by: (1) fermentation and extraction of broths from soil, plant or marine microorganisms or (2) extraction of plants or marine organisms. Natural product libraries include polyketides, non-ribosomal peptides, and variants (non-naturally occurring) thereof. For a review, see Science 282: 63-68 (1998). Combinatorial libraries are composed of large numbers of peptides, oligonucleotides, or organic compounds as a mixture. These libraries are relatively easy to prepare by traditional automated synthesis methods, PCR, cloning, or proprietary synthetic methods. Of particular interest are non-peptide combinatorial libraries. Still other libraries of interest include peptide, protein, peptidomimetic, multiparallel synthetic collection, recombinatorial, and polypeptide libraries. For a review of combinatorial chemistry and libraries created therefrom, see Myers, Curr. Opin. Biotechnol. 8: 701-707 (1997). Identification ofmodulators through use of the various libraries described herein permits modification of the candidate "hit" (or "lead") to optimize the capacity of the "hit" to modulate activity. Compound libraries may be purchased commercially (e.g., such as LeadQuest™-libraries from Tripos (St. Louis, MO.)) or may be synthesized using methods well known in the art.

Methods can be used to screen for antisense molecules that inhibit the functional expression of one or more mRNA molecules that modulate MHC Class I molecule expression. An antisense nucleic acid molecule may be constructed in a number of different ways provided that it is capable of interfering with the expression of a target protein. Typical antisense oligonucleotides to be screened preferably are 30-100 nucleotides in length. The antisense nucleic acid molecule generally will be substantially identical (although in antisense orientation) to the target MHC Class I molecule sequence. The minimal identity will typically be greater than about 80%, greater than about 90%, greater than about 95% or about 100% identical.

Nucleic acid modulators also may include ribozymes. Thus, methods can be used to screen for ribozyme molecules that inhibit the functional expression of one or more mRNA molecules that encode one or more proteins that modulate MHC Class I molecules. The design and use of target RNA-specific ribozymes is described in Haseloff et al., Nature 334: 585, 1988; see also U.S. Patent No. 5,646,023, for example. Tablor, et al., Gene 108: 175, 1991, have greatly simplified the construction of catalytic RNAs by combining the advantages of the anti-sense RNA and the ribozyme technologies in a single construct. Smaller regions of homology are required for ribozyme catalysis, therefore this can promote the repression of different members of a large gene family (e.g., Ig family) if the cleavage sites are conserved.

In another preferred embodiment, siRNAs are used to down-regulate, for example, MHC Class I molecules that have been identified as playing a role in a neural disorder. Several methods are available for the construction of siRNAs, including commercial available sources. siRNAs can be constructed using T7 phage polymerase. T7 polymerase is used to transcribe individual siRNA sense and antisense strands, which are then annealed to produce a siRNA. The T7 polymerase can also be used to transcribe siRNA strands that are linked in *cis*, forming a hairpin structure. The transcribed RNAs are comprised of 5' triphosphate termini or most preferred for a mammalian cell, 5' monophosphates. Successful siRNA-mediated knockdown of mammalian genes has been recently reported.

Another technique for drug screening provides for high throughput screening of compounds having suitable binding affinity to the protein of interest (see, e.g., Geysen et al., 1984, PCT application WO84/03564). In this method, large numbers of different small test compounds are synthesized on a solid substrate. The test compounds are reacted with MHC Class I molecules, or fragments thereof, and washed. A bound MHC Class I molecule is then detected by methods well known in the art. A purified MHC Class I molecule can also be coated directly onto plates for use in the aforementioned drug screening techniques. Alternatively, non-neutralizing antibodies can be used to capture the peptide and immobilize it on a solid support.

Diagnostic and research reagent kits are also provided which include components to determine identity of the MHC Class I molecule in a patient or other test subject. Thus, the kit may contain a sample of the MHC Class I molecule, gene, an allele or fragment thereof, or expression product of the MHC Class I molecule, gene, an allele or fragment thereof. The kit also may contain instructions (written) for conducting the diagnostic assay. The kit also may contain an assay or test support, typically a solid support, and other materials such as positive control samples, negative control samples, cells, enzymes, detection labels, buffers, etc.

### Assays for Identifying Candidate Agents having PirB Antagonist Activity:

The term "agent" or "compound" as used herein describes any molecule, e.g. protein or pharmaceutical, with the capability of antagonizing the biological activity of PirB. Generally a plurality of assay mixtures can be run in parallel with different agent concentrations to obtain a differential response to the various concentrations. Typically, one of these concentrations serves as a negative control, i.e. at zero concentration or below the level of detection.

Candidate agents (compounds) encompass numerous chemical classes, though typically they are organic molecules, preferably small organic compounds having a molecular weight of more than 50 and less than about 2,500 daltons. Candidate agents comprise functional groups necessary for structural interaction with proteins, particularly hydrogen bonding, and typically include at least an amine, carbonyl, hydroxyl or carboxyl group, preferably at least two of the functional chemical groups. The candidate agents often comprise cyclical carbon or heterocyclic structures and/or aromatic or polyaromatic structures substituted with one or more of the above functional groups. Candidate agents are also found among biomolecules including, but not limited to: peptides, saccharides, fatty acids, steroids, purines, pyrimidines, derivatives, structural analogs or combinations thereof.

Candidate agents are obtained from a wide variety of sources including libraries of synthetic or natural compounds. For example, numerous means are available for random and directed synthesis of a wide variety of organic compounds and biomolecules, including expression of randomized oligonucleotides and oligopeptides. Alternatively, libraries of natural compounds in the form of bacterial, fungal, plant and animal extracts are available or readily produced. Additionally, natural or synthetically produced libraries and compounds are readily modified through conventional chemical, physical and biochemical means, and may be used to produce combinatorial libraries. Known pharmacological agents may be subjected to directed or random chemical modifications, such as acylation, alkylation, esterification, amidification, etc. to produce structural analogs. Screening may be directed to known pharmacologically active compounds and chemical analogs thereof.

Where the screening assay is a binding assay utilizing the PirB receptor, one or more of the molecules may be joined to a label, where the label can directly or indirectly provide a detectable signal. Various labels include radioisotopes, fluorescers, chemiluminescers, enzymes, specific binding molecules, particles, e.g. magnetic particles, and the like. Specific binding molecules include pairs, such as biotin and streptavidin, digoxin and antidigoxin etc. For the specific binding members, the complementary member would normally be labeled with a molecule that provides for detection, in accordance with known procedures.

A variety of other reagents may be included in the screening assay. These include reagents like salts, neutral proteins, e.g. albumin, detergents, etc that are used to facilitate optimal protein-protein binding and/or reduce non-specific or background interactions. Reagents that improve the efficiency of the assay, such as protease inhibitors, nuclease inhibitors, anti-microbial agents, etc. may be used. The mixture of components are added in any order that provides for the requisite binding. Incubations are performed at any suitable temperature, typically between 4 and 40.degree. C. Incubation periods are selected for optimum activity, but may also be optimized to facilitate rapid high-throughput screening.

Also disclosed is a method for identifying a composition which binds to PirB or blocks KSHV env-mediated membrane fusion. The method includes incubating components comprising the composition and PirB under conditions sufficient to allow the components to interact and measuring the binding of the composition to PirB. Compositions that bind to PirB include peptides, peptidomimetics, polypeptides, chemical compounds and biologic agents as described above.

Incubating includes conditions which allow contact between the test composition and PirB. Binding can be measured indirectly by biochemical alterations in the cell (e.g., calcium flux). Contacting includes in solution and in solid phase. The test ligand(s)/composition may optionally be a combinatorial library for screening a plurality of compositions. Compositions identified in the method of the invention can be further evaluated, detected, cloned, sequenced, and the like, either in solution or after binding to a solid support, by any method usually applied to the detection of a specific DNA sequence such as PCR, oligomer restriction (Saiki, et al., Bio/Technology, 3:1008-1012, 1985), allele-specific oligonucleotide (ASO) probe analysis (Conner, et al., Proc. Natl. Acad. Sci. USA, 80:278, 1983), oligonucleotide ligation assays (OLAs) (Landegren, et al, Science, 241:1077, 1988), and the like. Molecular techniques for DNA analysis have been reviewed (Landegren, et al., Science, 242:229-237, 1988).

Any of a variety of procedures may be used to clone the genes of the present disclosure when the test composition is in a combinatorial library or is expressed as a gene product (as opposed to a chemical composition). One such method entails analyzing a shuttle vector library of DNA inserts (derived from a cell which expresses the composition) for the presence of an insert which contains the composition gene. Such an analysis may be conducted by transfecting cells with the vector and then assaying for expression of the composition binding activity. The preferred method for cloning these genes entails determining the amino acid sequence of the composition protein. Usually this task will be accomplished by purifying the desired composition protein and analyzing it with automated sequencers. Alternatively, each protein may be fragmented as with cyanogen bromide, or with proteases such as papain, chymotrypsin or trypsin (Oike, Y., et al., J. Biol. Chem., 257:9751-9758 (1982); Liu, C., et al., Int. J. Pept. Protein Res., 21:209-215 (1983)). Although it is possible to determine the entire amino acid sequence of these proteins, it is preferable to determine the sequence of peptide fragments of these molecules.

To determine if a composition can functionally complex with the receptor protein, induction of the exogenous gene is monitored by monitoring changes in the protein levels of the protein encoded for by the exogenous gene, for example. When a composition(s) is found that can induce transcription of the exogenous gene, it is concluded that this composition(s) can bind to the receptor protein coded for by the nucleic acid encoding the initial sample test composition(s).

Expression of the exogenous gene can be monitored by a functional assay or assay for a protein product, for example. The exogenous gene is therefore a gene which will provide an assayable/measurable expression product in order to allow detection of expression of the exogenous gene. Such exogenous genes include, but are not limited to, reporter genes such as chloramphenicol acetyltransferase gene, an alkaline phosphatase gene, beta-galactosidase,a luciferase gene, a green fluorescent protein gene, guanine xanthine phosphoribosyltransferase, alkaline phosphatase, and antibiotic resistance genes (e.g., neomycin phosphotransferase).

Expression of the exogenous gene is indicative of composition-receptor binding, thus, the binding or blocking composition can be identified and isolated. The compositions can be extracted and purified from the culture media or a cell by using known protein purification techniques commonly employed, such as extraction, precipitation, ion exchange chromatography, affinity chromatography, gel filtration and the like. Compositions can be isolated by affinity chromatography using the modified receptor protein extracellular domain bound to a column matrix or by heparin chromatography.

### PirB Antibodies

The present disclosure provides for antibodies against PirB that block PirB activation by binding to the PirB receptor, itself, or a PirB soluble ligand. Such antibodies are useful as research and diagnostic tools in the study of PirB associated disorders or diseases and the development of effective therapeutics. In addition, pharmaceutical compositions comprising antibodies against PirB may represent effective therapeutics.

Antibodies for use in the invention include polyclonal antibodies, monoclonal antibodies, and fragments of polyclonal and monoclonal antibodies.

The PirB polypeptides can also be used to produce antibodies which are immunoreactive or bind to epitopes of the PirB polypeptides. Antibody which consists essentially of pooled monoclonal antibodies with different epitopic specificities, as well as distinct monoclonal antibody preparations are provided. Monoclonal antibodies are made from antigen containing fragments of the protein by methods well known in the art (Kohler, et al., Nature, 256:495, 1975; Current Protocols in Molecular Biology, Ausubel, et al., ed., 1989).

The term "antibody" as used in this invention includes intact molecules as well as fragments thereof, such as Fab, F(ab')₂, and Fv which are capable of binding the epitopic determinant. These antibody fragments retain some ability to selectively bind with its antigen or receptor and are defined as follows:
(1) Fab, the fragment which contains a monovalent antigen-binding fragment of an antibody molecule can be produced by digestion of whole antibody with the enzyme papain to yield an intact light chain and a portion of one heavy chain;
(2) Fab', the fragment of an antibody molecule can be obtained by treating whole antibody with pepsin, followed by reduction, to yield an intact light chain and a portion of the heavy chain; two Fab' fragments are obtained per antibody molecule;
(3) (Fab')₂, the fragment of the antibody that can be obtained by treating whole antibody with the enzyme pepsin without subsequent reduction; F(ab')₂ is a dimer of two Fab' fragments held together by two disulfide bonds;
(4) Fv, defined as a genetically engineered fragment containing the variable region of the light chain and the variable region of the heavy chain expressed as two chains; and
(5) Single chain antibody ("SCA"), defined as a genetically engineered molecule containing the variable region of the light chain, the variable region of the heavy chain, linked by a suitable polypeptide linker as a genetically fused single chain molecule.

Methods of making these fragments are known in the art. (See for example, Harlow and Lane, Antibodies: A Laboratory Manual, Cold Spring Harbor Laboratory, New York (1988)).

As used in this invention, the term "epitope" means any antigenic determinant on an antigen to which the paratope of an antibody binds. Epitopic determinants usually consist of chemically active surface groupings of molecules such as amino acids or sugar side chains and usually have specific three dimensional structural characteristics, as well as specific charge characteristics.

Antibodies which bind to the PirB polypeptide can be prepared using an intact polypeptide or fragments containing small peptides of interest as the immunizing antigen. The polypeptide or a peptide used to immunize an animal can be derived from translated cDNA or chemical synthesis which can be conjugated to a carrier protein, if desired. Such commonly used carriers which are chemically coupled to the peptide include keyhole limpet hemocyanin (KLH), thyroglobulin, bovine serum albumin (BSA), and tetanus toxoid. The coupled peptide is then used to immunize the animal (e.g., a mouse, a rat, or a rabbit).

If desired, polyclonal or monoclonal antibodies can be further purified, for example, by binding to and elution from a matrix to which the polypeptide or a peptide to which the antibodies were raised is bound. Those of skill in the art will know of various techniques common in the immunology arts for purification and/or concentration of polyclonal antibodies, as well as monoclonal antibodies (See for example, Coligan, et al., Unit 9, Current Protocols in Immunology, Wiley Interscience, 1994).

It is also possible to use the anti-idiotype technology to produce monoclonal antibodies which mimic an epitope. For example, an anti-idiotypic monoclonal antibody made to a first monoclonal antibody will have a binding domain in the hypervariable region which is the "image" of the epitope bound by the first monoclonal antibody.

The preparation of polyclonal antibodies is well-known to those skilled in the art. See, for example, Green et al., Production of polyclonal Antisera, in IMMUNOCHEMICAL PROTOCOLS (Manson, ed.), pages 1-5 (Humana Press 1992); Coligan et al., Production of Polyclonal Antisera in Rabbits, Rats, Mice and Hamsters, in CURRENT PROTOCOLS IN IMMUNOLOGY, section 2.4.1 (1992).

The preparation of monoclonal antibodies likewise is conventional. See, for example, Kohler & Milstein, Nature 256:495 (1975); Coligan et al., sections 2.5.1-2.6.7; and Harlow et al., ANTIBODIES: A LABORATORY MANUAL, page 726 (Cold Spring Harbor Pub. 1988). Briefly, monoclonal antibodies can be obtained by injecting mice with a composition comprising an antigen, verifying the presence of antibody production by removing a serum sample, removing the spleen to obtain B lymphocytes, fusing the B lymphocytes with myeloma cells to produce hybridomas, cloning the hybridomas, selecting positive clones that produce antibodies to the antigen, and isolating the antibodies from the hybridoma cultures. Monoclonal antibodies can be isolated and purified from hybridoma cultures by a variety of well-established techniques. Such isolation techniques include affinity chromatography with Protein-A Sepharose, size-exclusion chromatography, and ion-exchange chromatography. See, e.g., Coligan et al., sections 2.7.1-2.7.12 and sections 2.9.1-2.9.3; Barnes et al., Purification of Immunoglobulin G (IgG), in METHODS IN MOLECULAR BIOLOGY, VOL. 10, pages 79-104 (Humana Press 1992). Methods of in vitro and in vivo multiplication of monoclonal antibodies is well-known to those skilled in the art. Multiplication in vitro may be carried out in suitable culture media such as Dulbecco's Modified Eagle Medium or RPMI 1640 medium, optionally replenished by a mammalian serum such as fetal calf serum or trace elements and growth-sustaining supplements such as normal mouse peritoneal exudate cells, spleen cells, bone marrow macrophages. Production in vitro provides relatively pure antibody preparations and allows scale-up to yield large amounts of the desired antibodies. Large scale hybridoma cultivation can be carried out by homogenous suspension culture in an airlift reactor, in a continuous stirrer reactor, or in immobilized or entrapped cell culture. Multiplication in vivo may be carried out by injecting cell clones into mammals histocompatible with the parent cells, e.g., syngeneic mice, to cause growth of antibody-producing tumors. Optionally, the animals are primed with a hydrocarbon, especially oils such as pristane (tetramethylpentadecane) prior to injection. After one to three weeks, the desired monoclonal antibody is recovered from the body fluid of the animal.

Therapeutic applications are conceivable for the antibodies. For example, antibodies may also be derived from subhuman primate antibody. General techniques for raising therapeutically useful antibodies in baboons may be found, for example, in Goldenberg et al., International Patent Publication WO 91/11465 (1991) and Losman et al., Int. J. Cancer 46:310 (1990).

Alternatively, a therapeutically useful anti-PirB antibody may be derived from a "humanized" monoclonal antibody. Humanized monoclonal antibodies are produced by transferring mouse complementary determining regions from heavy and light variable chains of the mouse immunoglobulin into a human variable domain, and then substituting human residues in the framework regions of the murine counterparts. The use of antibody components derived from humanized monoclonal antibodies obviates potential problems associated with the immunogenicity of murine constant regions. General techniques for cloning murine immunoglobulin variable domains are described, for example, by Orlandi et al., Proc. Nat'l Acad. Sci. USA 86:3833 (1989). Techniques for producing humanized monoclonal antibodies are described, for example, by Jones et al., Nature 321: 522 (1986); Riechmann et al., Nature 332: 323 (1988); Verhoeyen et al., Science 239: 1534 (1988); Carter et al., Proc. Nat'l Acad. Sci. USA 89: 4285 (1992); Sandhu, Crit. Rev. Biotech. 12: 437 (1992); and Singer et al., J. Immunol. 150: 2844 (1993).

Antibodies also may be derived from human antibody fragments isolated from a combinatorial immunoglobulin library. See, for example, Barbas et al., METHODS: A COMPANION TO METHODS IN ENZYMOLOGY, VOL.2, page 119 (1991); Winter et al.,Ann. Rev. Immunol. 12: 433 (1994). Cloning and expression vectors that are useful for producing a human immunoglobulin phage library can be obtained, for example, from STRATAGENE Cloning Systems (La Jolla, Calif.).

In addition, antibodies may be derived from a human monoclonal antibody. Such antibodies are obtained from transgenic mice that have been "engineered" to produce specific human antibodies in response to antigenic challenge. In this technique, elements of the human heavy and light chain loci are introduced into strains of mice derived from embryonic stem cell lines that contain targeted disruptions of the endogenous heavy and light chain loci. The transgenic mice can synthesize human antibodies specific for human antigens, and the mice can be used to produce human antibody-secreting hybridomas. Methods for obtaining human antibodies from transgenic mice are described by Green et al., Nature Genet. 7:13 (1994); Lonberg et al., Nature 368:856 (1994); and Taylor et al., Int. Immunol. 6:579 (1994).

Antibody fragments can be prepared by proteolytic hydrolysis of the antibody or by expression in E. Coli of DNA encoding the fragment. Antibody fragments can be obtained by pepsin or papain digestion of whole antibodies by conventional methods. For example, antibody fragments can be produced by enzymatic cleavage of antibodies with pepsin to provide a 5S fragment denoted F(ab')₂. This fragment can be further cleaved using a thiol reducing agent, and optionally a blocking group for the sulfhydryl groups resulting from cleavage of disulfide linkages, to produce 3.5S Fab' monovalent fragments. Alternatively, an enzymatic cleavage using papain produces two monovalent Fab fragments and an Fc fragment directly. These methods are described, for example, by Goldenberg, U.S. Pat. Nos. 4,036,945 and 4,331,647, and references contained therein. See also Nisonhoff et al., Arch. Biochem. Biophys. 89:230 (1960); Porter, Biochem. J. 73:119 (1959); Edelman et al., METHODS IN ENZYMOLOGY, VOL. 1, page 422 (Academic Press 1967); and Coligan et al. at sections 2.8.1-2.8.10 and 2.10.1-2.10.4.

Other methods of cleaving antibodies, such as separation of heavy chains to form monovalent light-heavy chain fragments, further cleavage of fragments, or other enzymatic, chemical, or genetic techniques may also be used, so long as the fragments bind to the antigen that is recognized by the intact antibody.

For example, Fv fragments comprise an association of V_{H} and V_{L} chains. This association may be noncovalent, as described in Inbar et al., Proc. Nat'l Acad. Sci. USA 69:2659 (1972). Alternatively, the variable chains can be linked by an intermolecular disulfide bond or cross-linked by chemicals such as glutaraldehyde. See, e.g., Sandhu, supra. Preferably, the Fv fragments comprise V and V_{L} chains connected by a peptide linker. These single-chain antigen binding proteins (sFv) are prepared by constructing a structural gene comprising DNA sequences encoding the V and V_{L} domains connected by an oligonucleotide. The structural gene is inserted into an expression vector, which is subsequently introduced into a host cell such as E. coli. The recombinant host cells synthesize a single polypeptide chain with a linker peptide bridging the two V domains. Methods for producing sFvs are described, for example, by Whitlow et al., METHODS: A COMPANION TO METHODS IN ENZYMOLOGY, VOL. 2, page 97 (1991); Bird et al., Science 242:423-426 (1988); Ladner et al., U.S. Pat. No. 4,946,778; Pack et al., Bio/Technology 11: 1271-77 (1993); and Sandhu, supra.

Another form of an antibody fragment is a peptide coding for a single complementarity-determining region (CDR). CDR peptides ("minimal recognition units") can be obtained by constructing genes encoding the CDR of an antibody of interest. Such genes are prepared, for example, by using the polymerase chain reaction to synthesize the variable region from RNA of antibody-producing cells. See, for example, Larrick et al., METHODS: A COMPANION TO METHODS IN ENZYMOLOGY, VOL. 2, page 106 (1991).

### Peptide Fragments of PirB :

The present disclosure also relates to substantially purified peptide fragments of PirB that block activation of PirB. Such peptide fragments could represent research and diagnostic tools in the study of PirB associated therapeutics. In addition, pharmaceutical compositions comprising isolated and purified peptide fragments of PirB may represent effective therapeutics.

The term "substantially purified" as used herein refers to a molecule, such as a peptide that is substantially free of other proteins, lipids, carbohydrates, nucleic acids, and other biological materials with which it is naturally associated. For example, a substantially pure molecule, such as a polypeptide, can be at least 60%, by dry weight, the molecule of interest. One skilled in the art can purify PIRB peptides using standard protein purification methods and the purity of the polypeptides can be determined using standard methods including, e.g., polyacrylamide gel electrophoresis (e.g., SDS-PAGE), column chromatography (e.g., high performance liquid chromatography (HPLC)), and amino-terminal amino acid sequence analysis.

Not only fragments of naturally-occurring PirB are disclosed, but also to PirB mutants and chemically synthesized derivatives of PirB that block the receptor's activation.

For example, changes in the amino acid sequence of PirB are contemplated. PirB can be altered by changing the DNA encoding the protein. Preferably, only conservative amino acid alterations are undertaken, using amino acids that have the same or similar properties. Illustrative amino acid substitutions include the changes of: alanine to serine; arginine to lysine; asparagine to glutamine or histidine; aspartate to glutamate; cysteine to serine; glutamine to asparagine; glutamate to aspartate; glycine to proline; histidine to asparagine or glutamine; isoleucine to leucine or valine; leucine to valine or isoleucine; lysine to arginine, glutamine, or glutamate; methionine to leucine or isoleucine; phenylalanine to tyrosine, leucine or methionine; serine to threonine; threonine to serine; tryptophan to tyrosine; tyrosine to tryptophan or phenylalanine; valine to isoleucine or leucine

Additionally, other variants and fragments of PIRB can be used. Variants include analogs, homologs, derivatives, muteins and mimetics of PIRB that retain the ability to block membrane fusion. Fragments of the PirB refer to portions of the amino acid sequence of PirB that also retain this ability. The variants and fragments can be generated directly from PirB itself by chemical modification, by proteolytic enzyme digestion, or by combinations thereof. Additionally, genetic engineering techniques, as well as methods of synthesizing polypeptides directly from amino acid residues, can be employed.

Non-peptide compounds that mimic the antagonistic binding of PirB ("mimetics") can be produced by the approach outlined in Saragovi et al., Science 253: 792-95 (1991). Mimetics are molecules which mimic elements of protein secondary structure. See, for example, Johnson et al.,"Peptide Turn Mimetics," in BIOTECHNOLOGY AND PHARMACY, Pezzuto et al., Eds., (Chapman and Hall, New York 1993). The underlying rationale behind the use of peptide mimetics is that the peptide backbone of proteins exists chiefly to orient amino acid side chains in such a way as to facilitate molecular interactions. For the purposes of the present invention, appropriate mimetics can be considered to be the equivalent of PirB itself when administered to bind PirB ligands.

Variants and fragments also can be created by recombinant techniques employing genomic or cDNA cloning methods. Site-specific and region-directed mutagenesis techniques can be employed. See CURRENT PROTOCOLS IN MOLECULAR BIOLOGY vol. 1, ch. 8 (Ausubel et al. eds., J. Wiley & Sons 1989 & Supp. 1990-93); PROTEIN ENGINEERING (Oxender & Fox eds., A. Liss, Inc. 1987). In addition, linker-scanning and PCR-mediated techniques can be employed for mutagenesis. See PCR TECHNOLOGY (Erlich ed., Stockton Press 1989); CURRENT PROTOCOLS IN MOLECULAR BIOLOGY, vols. 1 & 2, supra. Protein sequencing, structure and modeling approaches for use with any of the above techniques are disclosed in PROTEIN ENGINEERING, loc. cit., and CURRENT PROTOCOLS IN MOLECULAR BIOLOGY, vols. 1 & 2, supra.

### Inhibiting PirB Expression:

There are several methods which can be employed in the inhibition or reduction of PirB Gene expression. these include:
"Gene silencing" refers to the suppression of gene expression, e.g., transgene, heterologous gene and/or endogenous gene expression. Gene silencing may be mediated through processes that affect transcription and/or through processes that affect post-transcriptional mechanisms. In some embodiments, gene silencing occurs when siRNA initiates the degradation of the mRNA of a gene of interest in a sequence-specific manner via RNA interference. In some embodiments, gene silencing may be allele-specific. "Allele-specific" gene silencing refers to the specific silencing of one allele of a gene.
"Knock-down," "knock-down technology" refers to a technique of gene silencing in which the expression of a target gene is reduced as compared to the gene expression prior to the introduction of the siRNA, which can lead to the inhibition of production of the target gene product. The term "reduced" is used herein to indicate that the target gene expression is lowered by 1-100%. For example, the expression may be reduced by 10, 20, 30, 40, 50, 60, 70, 80, 90, 95, or even 99%. Knock-down of gene expression can be directed by the use of dsRNAs or siRNAs. For example, "RNA interference (RNAi)," which can involve the use of siRNA, has been successfully applied to knockdown the expression of specific genes in plants, D. melanogaster, C. elegans, trypanosomes, planaria, hydra, and several vertebrate species including the mouse.
"RNA interference (RNAi)" is the process of sequence-specific, post-transcriptional gene silencing initiated by siRNA. RNAi is seen in a number of organisms such as Drosophila, nematodes, fungi and plants, and is believed to be involved in antiviral defense, modulation of transposon activity, and regulation of gene expression. During RNAi, siRNA induces degradation of target mRNA with consequent sequence-specific inhibition of gene expression.
A "small interfering" or "short interfering RNA" or siRNA is a RNA duplex of nucleotides that is targeted to a gene interest. A "RNA duplex" refers to the structure formed by the complementary pairing between two regions of a RNA molecule. siRNA is "targeted" to a gene in that the nucleotide sequence of the duplex portion of the siRNA is complementary to a nucleotide sequence of the targeted gene. In some embodiments, the length of the duplex of siRNAs is less than 30 nucleotides. In some embodiments, the duplex can be 29, 28, 27, 26, 25, 24, 23, 22, 21, 20, 19, 18, 17, 16, 15, 14, 13, 12, 11 or 10 nucleotides in length. In some embodiments, the length of the duplex is 19-25 nucleotides in length. The RNA duplex portion of the siRNA can be part of a hairpin structure. In addition to the duplex portion, the hairpin structure may contain a loop portion positioned between the two sequences that form the duplex. The loop can vary in length. In some embodiments the loop is 5, 6, 7, 8, 9, 10, 11, 12 or 13 nucleotides in length. The hairpin structure can also contain 3' or 5' overhang portions. In some embodiments, the overhang is a 3' or a 5' overhang 0, 1, 2, 3, 4 or 5 nucleotides in length.

Furthermore, the term "short interfering RNA", "siRNA", "short interfering nucleic acid molecule", "short interfering oligonucleotide molecule", or "chemically-modified short interfering nucleic acid molecule" as used herein refers to any nucleic acid molecule capable of inhibiting or down regulating gene expression or viral replication, for example by mediating RNA interference "RNAi" or gene silencing in a sequence-specific manner; see for example Zamore et al., 2000, Cell, 101, 25-33; Bass, 2001, Nature, 411, 428-429; Elbashir et al., 2001, Nature, 411, 494-498; and Kreutzer et al., International PCT Publication No. WO 00/44895; Zernicka-Goetz et al., International PCT Publication No. WO 01/36646; Fire, International PCT Publication No. WO 99/32619; Plaetinck et al., International PCT Publication No. WO 00/01846; Mello and Fire, International PCT Publication No. WO O1/29058; Deschamps-Depaillette, International PCT Publication No. WO 99/07409; and Li et al., International PCT Publication No. WO 00/44914; Allshire, 2002, Science, 297, 1818-1819; Volpe et al., 2002, Science, 297, 1833-1837; Jenuwein, 2002, Science, 297, 2215-2218; and Hall et al., 2002, Science, 297, 2232-2237; Hutvagner and Zamore, 2002, Science, 297, 2056-60; McManus et al., 2002, RNA, 8, 842-850; Reinhart et al., 2002, Gene & Dev., 16, 1616-1626; and Reinhart & Bartel, 2002, Science, 297, 1831). Non limiting examples of siRNA molecules of the invention are shown in FIGS. 18-20, and Table I herein. For example the siRNA can be a double-stranded polynucleotide molecule comprising self-complementary sense and antisense regions, wherein the antisense region comprises nucleotide sequence that is complementary to nucleotide sequence in a target nucleic acid molecule or a portion thereof and the sense region having nucleotide sequence corresponding to the target nucleic acid sequence or a portion thereof. The siRNA can be assembled from two separate oligonucleotides, where one strand is the sense strand and the other is the antisense strand, wherein the antisense and sense strands are self-complementary (i.e. each strand comprises nucleotide sequence that is complementary to nucleotide sequence in the other strand; such as where the antisense strand and sense strand form a duplex or double stranded structure, for example wherein the double stranded region is about 19 base pairs); the antisense strand comprises nucleotide sequence that is complementary to nucleotide sequence in a target nucleic acid molecule or a portion thereof and the sense strand comprises nucleotide sequence corresponding to the target nucleic acid sequence or a portion thereof. Alternatively, the siRNA is assembled from a single oligonucleotide, where the self-complementary sense and antisense regions of the siRNA are linked by means of a nucleic acid based or non-nucleic acid-based linker(s). The siRNA can be a polynucleotide with a duplex, asymmetric duplex, hairpin or asymmetric hairpin secondary structure, having self-complementary sense and antisense regions, wherein the antisense region comprises nucleotide sequence that is complementary to nucleotide sequence in a separate target nucleic acid molecule or a portion thereof and the sense region having nucleotide sequence corresponding to the target nucleic acid sequence or a portion thereof. The siRNA can be a circular single-stranded polynucleotide having two or more loop structures and a stem comprising self-complementary sense and antisense regions, wherein the antisense region comprises nucleotide sequence that is complementary to nucleotide sequence in a target nucleic acid molecule or a portion thereof and the sense region having nucleotide sequence corresponding to the target nucleic acid sequence or a portion thereof, and wherein the circular polynucleotide can be processed either in vivo or in vitro to generate an active siRNA molecule capable of mediating RNAi. The siRNA can also comprise a single stranded polynucleotide having nucleotide sequence complementary to nucleotide sequence in a target nucleic acid molecule or a portion thereof (for example, where such siRNA molecule does not require the presence within the siRNA molecule of nucleotide sequence corresponding to the target nucleic acid sequence or a portion thereof), wherein the single stranded polynucleotide can further comprise a terminal phosphate group, such as a 5'-phosphate (see for example Martinez et al., 2002, Cell., 110, 563-574 and Schwarz et al., 2002, Molecular Cell, 10, 537-568), or 5',3'-diphosphate. In certain embodiment, the siRNA molecule of the invention comprises separate sense and antisense sequences or regions, wherein the sense and antisense regions are covalently linked by nucleotide or non-nucleotide linkers molecules as is known in the art, or are alternately non-covalently linked by ionic interactions, hydrogen bonding, van der waals interactions, hydrophobic intercations, and/or stacking interactions. In certain embodiments, the siRNA molecules of the invention comprise nucleotide sequence that is complementary to nucleotide sequence of a target gene. In another embodiment, the siRNA molecule of the invention interacts with nucleotide sequence of a target gene in a manner that causes inhibition of expression of the target gene. As used herein, siRNA molecules need not be limited to those molecules containing only RNA, but further encompasses chemically-modified nucleotides and non-nucleotides. In certain embodiments, the short interfering nucleic acid molecules of the invention lack 2'-hydroxy (2'-OH) containing nucleotides. Optionally, siRNA molecules can comprise ribonucleotides at about 5, 10, 20, 30, 40, or 50% of the nucleotide positions. The modified short interfering nucleic acid molecules of the invention can also be referred to as short interfering modified oligonucleotides "siMON." As used herein, the term siRNA is meant to be equivalent to other terms used to describe nucleic acid molecules that are capable of mediating sequence specific RNAi, for example short interfering RNA (siRNA), double-stranded RNA (dsRNA), micro-RNA (miRNA), short hairpin RNA (shRNA), short interfering oligonucleotide, short interfering nucleic acid, short interfering modified oligonucleotide, chemically-modified siRNA, post-transcriptional gene silencing RNA (ptgsRNA), and others. In addition, as used herein, the term RNAi is meant to be equivalent to other terms used to describe sequence specific RNA interference, such as post transcriptional gene silencing, translational inhibition, or epigenetics. For example, siRNA molecules can be used to epigenetically silence genes at both the post-transcriptional level or the pre-transcriptional level. In a non-limiting example, epigenetic regulation of gene expression by siRNA molecules of the invention can result from siRNA mediated modification of chromatin structure to alter gene expression (see, for example, Verdel et al., 2004, Science, 303, 672-676; Pal-Bhadra et al., 2004, Science, 303, 669-672; Allshire, 2002, Science, 297, 1818-1819; Volpe et al., 2002, Science, 297, 1833-1837; Jenuwein, 2002, Science, 297, 2215-2218; and Hall et al., 2002, Science, 297, 2232-2237).

The siRNA can be encoded by a nucleic acid sequence, and the nucleic acid sequence can also include a promoter. The nucleic acid sequence can also include a polyadenylation signal. In some embodiments, the polyadenylation signal is a synthetic minimal polyadenylation signal.

### Synthesis of siRNA Molecules of the Invention

The method of synthesis used for RNA including certain siRNA molecules for use in the invention follows the procedure as described in Usman et al., 1987, J. Am. Chem. Soc., 109, 7845; Scaringe et al., 1990, Nucleic Acids Res., 18, 5433; and Wincott et al., 1995, Nucleic Acids Res. 23, 2677-2684 Wincott et al., 1997, Methods Mol. Bio., 74, 59, and makes use of common nucleic acid protecting and coupling groups, such as dimethoxytrityl at the 5'-end, and phosphoramidites at the 3'-end. In a non-limiting example, small scale syntheses are conducted on a 394 Applied Biosystems, Inc. synthesizer using a 0.2 .mu.mol scale protocol with a 7.5 min coupling step for alkylsilyl protected nucleotides and a 2.5 min coupling step for 2'-O-methylated nucleotides. Alternatively, syntheses at the 0.2 umol scale can be done on a 96-well plate synthesizer, such as the instrument produced by Protogene (Palo Alto, Calif.) with minimal modification to the cycle. A 33-fold excess (60 uL of 0.11 M=6.6 umol) of 2'-O-methyl phosphoramidite and a 75-fold excess of S-ethyl tetrazole (60 uL of 0.25 M=15 umol) can be used in each coupling cycle of 2'-O-methyl residues relative to polymer-bound 5'-hydroxyl. A 66-fold excess (120 uL of 0.11 M=13.2 umol) of alkylsilyl (ribo) protected phosphoramidite and a 150-fold excess of S-ethyl tetrazole (120 uL of 0.25 M=30 umol) can be used in each coupling cycle of ribo residues relative to polymer-bound 5'-hydroxyl. Average coupling yields on the 394 Applied Biosystems, Inc. synthesizer, determined by colorimetric quantitation of the trityl fractions, are typically 97.5-99%. Other oligonucleotide synthesis reagents for the 394 Applied Biosystems, Inc. synthesizer include the following: detritylation solution is 3% TCA in methylene chloride (ABI); capping is performed with 16% N-methyl imidazole in THF (ABI) and 10% acetic anhydride/10% 2,6-lutidine in TBF (ABI); oxidation solution is 16.9 mM I₂, 49 mM pyridine, 9% water in THF (PERSEPTIVE™). Burdick & Jackson Synthesis Grade acetonitrile is used directly from the reagent bottle. S-Ethyltetrazole solution (0.25 M in acetonitrile) is made up from the solid obtained from American International Chemical, Inc. Alternately, for the introduction of phosphorothioate linkages, Beaucage reagent (3H-1,2-Benzodithiol-3-one 1,1-dioxide 0.05 M in acetonitrile) is used.

Deprotection of the RNA is performed using either a two-pot or one-pot protocol. For the two-pot protocol, the polymer-bound trityl-on oligoribonucleotide is transferred to a 4 mL glass screw top vial and suspended in a solution of 40% aq. methylamine (1 mL) at 65degrees C for 10 minutes. After cooling to ―20 degrees C, the supernatant is removed from the polymer support. The support is washed three times with 1.0 mL of EtOH:MeCN:H2O/3:1:1, vortexed and the supernatant is then added to the first supernatant. The combined supernatants, containing the oligoribonucleotide, are dried to a white powder. The base deprotected oligoribonucleotide is resuspended in anhydrous TEA/HF/NMP solution (300 uL of a solution of 1.5 mL N-methylpyrrolidinone, 750 uL TEA and 1 mL TEA.3HF to provide a 1.4 M HF concentration) and heated to 65 degrees C After 1.5 h, the oligomer is quenched with 1.5 M NH₄HCO₃.

Alternatively, for the one-pot protocol, the polymer-bound trityl-on oligoribonucleotide is transferred to a 4 mL glass screw top vial and suspended in a solution of 33% ethanolic methylamine/DMSO: 1/1 (0.8 mL) at 65degrees C for 15 minutes. The vial is brought to room temperature TEA.3HF (0.1 mL) is added and the vial is heated at 65 degrees C for 15 minutes. The sample is cooled at ―20 degrees C and then quenched with 1.5 M NH₄HCO₃.

For purification of the trityl-on oligomers, the quenched NH₄HCO₃. solution is loaded onto a C-18 containing cartridge that had been prewashed with acetonitrile followed by 50 mM TEAA. After washing the loaded cartridge with water, the RNA is detritylated with 0.5% TFA for 13 minutes. The cartridge is then washed again with water, salt exchanged with 1 M NaCl and washed with water again. The oligonucleotide is then eluted with 30% acetonitrile.

The average stepwise coupling yields are typically >98% (Wincott et al., 1995 Nucleic Acids Res. 23, 2677-2684). Those of ordinary skill in the art will recognize that the scale of synthesis can be adapted to be larger or smaller than the example described above including but not limited to 96-well format.

Alternatively, the nucleic acid molecules for use in the present invention can be synthesized separately and joined together post-synthetically, for example, by ligation (Moore et al., 1992, Science 256, 9923; Draper et al., International PCT publication No. WO 93/23569; Shabarova et al., 1991, Nucleic Acids Research 19, 4247; Bellon et al., 1997, Nucleosides & Nucleotides, 16, 951; Bellon et al., 1997, Bioconjugate Chem. 8. 204), or by hybridization following synthesis and/or deprotection.

It will be apparent to one skilled in the art that the inclusion of modified bases, as well as the naturally occuring bases cytosine, uracil, adenosine and guanosine, may confer advantageous properties on siRNA molecules containing said modified bases. For example, modified bases may increase the stability of the siRNA molecule thereby reducing the amount required to produce a desired effect. The provision of modified bases may also provide siRNA molecules which are more or less stable.

The term "modified nucleotide base" encompasses nucleotides with a covalently modified base and/or sugar. For example, modified nucleotides include nucleotides having sugars which are covalently attached to low molecular weight organic groups other than a hydroxyl group at the 3' position and other than a phosphate group at the 5' position. Thus modified nucleotides may also include 2' substituted sugars such as 2'-O-methyl-; 2-O-alkyl; 2-O-allyl; 2'-S-alkyl; 2'-S-allyl; 2'-fluoro-; 2'-halo or 2;azido-ribose, carbocyclic sugar analogues a-anomeric sugars; epimeric sugars such as arabinose, xyloses or lyxoses, pyranose sugars, furanose sugars, and sedoheptulose.

Modified nucleotides are known in the art and include by example and not by way of limitation; alkylated purines and/or pyrimidines; acylated purines and/or pyrimidines; or other heterocycles. These classes of pyrimidines and purines are known in the art and include, pseudoisocytosine; N4, N4-ethanocytosine; 8-hydroxy-N6-methyladenine; 4-acetylcytosine, 5-(carboxyhydroxylmethyl) uracil; 5-fluorouracil; 5-bromouracil; 5-carboxymethylaminomethyl-2-thiouracil; 5-carboxymethylaminomethyl uracil; dihydrouracil; inosine; N6-isopentyl-adenine; 1-methyladenine; 1-methylpseudouracil; 1-methylguanine; 2,2-dimethylguanine; 2-methyladenine; 2-methylguanine; 3-methylcytosine; 5-methylcytosine; N6-methyladenine; 7-methylguanine; 5-methylaminomethyl uracil; 5-methoxy amino methyl-2-thiouracil; .beta.-D-mannosylqueosine; 5-methoxycarbonylmethyluracil; 5-methoxyuracil; 2 methylthio-N-6-isopentenyladenine; uracil-5-oxyacetic acid methyl ester; psueouracil; 2-thiocytosine; 5-methyl-2 thiouracil, 2-thiouracil; 4-thiouracil; 5-methyluracil; N-uracil-5-oxyacetic acid methylester, uracil 5-oxyacetic acid; queosine; 2-thiocytosine; 5-propyluracil; 5-propylcytosine; 5-ethyluracil; 5-ethylcytosine; 5-butyluracil; 5-pentyluracil; 5-pentylcytosine; and 2,6,-diaminopurine; methylpsuedouracil; 1-methylguanine; 1-methylcytosine.

The siRNA molecules for use in the invention can be synthesized using conventional phosphodiester linked nucleotides and synthesized using standard solid or solution phase synthesis techniques which are known in the art. Linkages between nucleotides may use alternative linking molecules. For example, linking groups of the formula P (O) S, (thioate); P (S) S, (dithioate); P (O) NR12; P (O) R'; P (O) OR6; CO; or CONR12 wherein R is H (or a salt) or alkyl (1-12C) and R6 is alkyl (1-9C) is joined to adjacent nucleotides through --O-- or --S--.

### Administration of siRNA Moleclues of the Invention

A siRNA molecule for use in the invention can be adapted for use to treat any PirB associated disease or disorder, and other indications that can respond to the level of gene product in a cell or tissue, alone or in combination with other therapies. Non-limiting examples of such diseases and conditions include those as defined under Nervous System defects, disorders and diseases described above, and any other indications that can respond to the level of an expressed gene product in a cell or organsim (see for example McSwiggen, International PCT Publication No. WO 03/74654). For example, a siRNA molecule can comprise a delivery vehicle, including liposomes, for administration to a subject, carriers and diluents and their salts, and/or can be present in pharmaceutically acceptable formulations. Methods for the delivery of nucleic acid molecules are described in Akhtar et al., 1992, Trends Cell Bio., 2, 139; Delivery Strategies for Antisense Oligonucleotide Therapeutics, ed. Akhtar, 1995, Maurer et al., 1999, Mol. Membr. Biol., 16, 129-140; Hofland and Huang, 1999, Handb. Exp. Pharmacol., 137, 165-192; and Lee et al., 2000, ACS Symp. Ser., 752, 184-192. Beigelman et al., U.S. Pat. No. 6,395,713 and Sullivan et al., PCT WO 94/02595 further describe the general methods for delivery of nucleic acid molecules. These protocols can be utilized for the delivery of virtually any nucleic acid molecule. Nucleic acid molecules can be administered to cells by a variety of methods known to those of skill in the art, including, but not restricted to, encapsulation in liposomes, by iontophoresis, or by incorporation into other vehicles, such as biodegradable polymers, hydrogels, cyclodextrins (see for example Gonzalez et al., 1999, Bioconjugate Chem., 10, 1068-1074; Wang et al., International PCT publication Nos. WO 03/47518 and WO 03/46185), poly(lactic-co-glycolic)ac- id (PLGA) and PLCA microspheres (see for example U.S. Pat. No. 6,447,796 and US Patent Application Publication No. US 2002130430), biodegradable nanocapsules, and bioadhesive microspheres, or by proteinaceous vectors (O'Hare and Normand, International PCT Publication No. WO 00/53722). In one embodiment, nucleic acid molecules or the invention are administered via biodegradable implant materials, such as elastic shape memory polymers (see for example Lendelein and Langer, 2002, Science, 296, 1673). In another embodiment, the nucleic acid molecules of the invention can also be formulated or complexed with polyethyleneimine and derivatives thereof, such as polyethyleneimine-polyethyleneglycol-N-acety- 1galactosamine (PEI-PEG-GAL) or polyethyleneimine-polyethyleneglycol-tri-N- -acetylgalactosamine (PEI-PEG-triGAL) derivatives. Alternatively, the nucleic acid/vehicle combination is locally delivered by direct injection or by use of an infusion pump. Direct injection of the nucleic acid molecules of the invention, whether subcutaneous, intramuscular, or intradermal, can take place using standard needle and syringe methodologies, or by needle-free technologies such as those described in Conry et al., 1999, Clin. Cancer Res., 5, 2330-2337 and Barry et al., International PCT Publication No. WO 99/31262. Many examples in the art describe CNS delivery methods of oligonucleotides by osmotic pump, (see Chun et al., 1998, Neuroscience Letters, 257, 135-138, D'Aldin et al., 1998, Mol. Brain Research, 55, 151-164, Dryden et al., 1998, J. Endocrinol., 157, 169-175, Ghimikar et al., 1998, Neuroscience Letters, 247, 21-24) or direct infusion (Broaddus et al., 1997, Neurosurg. Focus, 3, article 4). Other routes of delivery include, but are not limited to oral (tablet or pill form) and/or intrathecal delivery (Gold, 1997, Neuroscience, 76, 1153-1158). More detailed descriptions of nucleic acid delivery and administration are provided in Sullivan et al., supra, Draper et al., PCT WO93/23569, Beigelman et al., PCT WO99/05094, and Klimuk et al. PCT W099/04819. The molecules for use in the instant invention can be used as pharmaceutical agents. Pharmaceutical agents prevent, modulate the occurrence, or treat (alleviate a symptom to some extent, preferably all of the symptoms) of a disease state in a subject.

A siRNA molecule can be complexed with membrane disruptive agents such as those described in U.S. Patent Appliaction Publication No. 20010007666. The membrane disruptive agent or agents and the siRNA molecule can also be complexed with a cationic lipid or helper lipid molecule, such as those lipids described in U.S. Pat. No. 6,235,310.

Alternatively, siRNA molecules for use in the invention are formulated or complexed with polyethylenimine (e.g., linear or branched PEI) and/or polyethylenimine derivatives, including for example grafted PEIs such as galactose PEI, cholesterol PEI, antibody derivatized PEI, and polyethylene glycol PEI (PEG-PEI) derivatives thereof (see for example Ogris et al., 2001, AAPA PharmSci, 3, 1-11; Furgeson et al., 2003, Bioconjugate Chem., 14, 840-847; Kunath et al., 2002, Phramaceutical Research, 19, 810-817; Choi et al., 2001, Bull. Korean Chem. Soc., 22, 46-52; Bettinger et al., 1999, Bioconjugate Chem., 10, 558-561; Peterson et al., 2002, Bioconjugate Chem., 13, 845-854; Erbacher et al., 1999, Journal of Gene Medicine Preprint, 1, 1-18; Godbey et al., 1999., PNAS USA, 96, 5177-5181; Godbey et al., 1999, Journal of Controlled Release, 60, 149-160; Diebold et al., 1999, Journal of Biological Chemistry, 274, 19087-19094; Thomas and Klibanov, 2002, PNAS USA, 99, 14640-14645; and Sagara, U.S. Pat. No. 6,586,524.

A siRNA molecule for use in the invention may comprise a bioconjugate, for example a nucleic acid conjugate as described in Vargeese et al., U.S. Ser. No. 10/427,160, filed Apr. 30,2003; U.S. Pat. No. 6,528,631; U.S. Pat. No. 6,335,434; U.S. Pat. No. 6,235,886; U.S. Pat. No. 6,153,737; U.S. Pat. No. 5,214,136; U.S. Pat. No. 5,138,045.

Thus, disclosed is a pharmaceutical composition comprising one or more nucleic acid(s) for use in the invention in an acceptable carrier, such as a stabilizer, buffer, and the like. The polynucleotides for use in the invention can be administered (e.g., RNA, DNA or protein) and introduced into a subject by any standard means, with or without stabilizers, buffers, and the like, to form a pharmaceutical composition. When it is desired to use a liposome delivery mechanism, standard protocols for formation of liposomes can be followed. The compositions can also be formulated and used as tablets, capsules or elixirs for oral administration, suppositories for rectal administration, sterile solutions, suspensions for injectable administration, and the other compositions known in the art.

Also disclosed are pharmaceutically acceptable formulations of the compounds described. These formulations include salts of the above compounds, e.g., acid addition salts, for example, salts of hydrochloric, hydrobromic, acetic acid, and benzene sulfonic acid.

A pharmacological composition or formulation refers to a composition or formulation in a form suitable for administration, e.g., systemic administration, into a cell or subject, including for example a human. Suitable forms, in part, depend upon the use or the route of entry, for example oral, transdermal, or by injection. Such forms should not prevent the composition or formulation from reaching a target cell (i.e., a cell to which the negatively charged nucleic acid is desirable for delivery). For example, pharmacological compositions injected into the blood stream should be soluble. Other factors are known in the art, and include considerations such as toxicity and forms that prevent the composition or formulation from exerting its effect.

By "systemic administration" is meant in vivo systemic absorption or accumulation of drugs in the blood stream followed by distribution throughout the entire body. Administration routes that lead to systemic absorption include, without limitation: intravenous, subcutaneous, intraperitoneal, inhalation, oral, intrapulmonary and intramuscular. Each of these administration routes exposes the siRNA molecules for use in the invention to an accessible diseased tissue. The rate of entry of a drug into the circulation has been shown to be a function of molecular weight or size. The use of a liposome or other drug carrier comprising the compounds for use in the instant invention can potentially localize the drug, for example, in certain tissue types, such as the tissues of the reticular endothelial system (RES). A liposome formulation that can facilitate the association of drug with the surface of cells, such as, lymphocytes and macrophages is also useful. This approach can provide enhanced delivery of the drug to target cells by taking advantage of the specificity of macrophage and lymphocyte immune recognition of abnormal cells, such as cancer cells.

By "pharmaceutically acceptable formulation" is meant a composition or formulation that allows for the effective distribution of the nucleic acid molecules of the instant invention in the physical location most suitable for their desired activity. Non-limiting examples of agents suitable for formulation with the nucleic acid molecules of the instant invention include: P-glycoprotein inhibitors (such as Pluronic P85), which can enhance entry of drugs into the CNS (Jolliet-Riant and Tillement, 1999, Fundam. Clin. Pharmacol., 13, 16-26); biodegradable polymers, such as poly (DL-lactide-coglycolide) microspheres for sustained release delivery after intracerebral implantation (Emerich, D F et al, 1999, Cell Transplant, 8, 47-58) (Alkermes, Inc. Cambridge, Mass.); and loaded nanoparticles, such as those made of polybutylcyanoacrylate, which can deliver drugs across the blood brain barrier and can alter neuronal uptake mechanisms (Prog Neuropsychopharmacol Biol Psychiatry, 23, 941-949, 1999). Other non-limiting examples of delivery strategies for the nucleic acid molecules for use in the instant invention include material described in Boado et al., 1998, J. Pharm. Sci., 87, 1308-1315; Tyler et al., 1999, FEBS Lett., 421, 280-284; Pardridge et al., 1995, PNAS USA., 92, 5592-5596; Boado, 1995, Adv. Drug Delivery Rev., 15, 73-107; Aldrian-Herrada et al., 1998, Nucleic Acids Res., 26, 4910-4916; and Tyler et al., 1999, PNAS USA., 96, 7053-7058.

Also disclosed is the use of the composition comprising surface-modified liposomes containing poly (ethylene glycol) lipids (PEG-modified, or long-circulating liposomes or stealth liposomes). These formulations offer a method for increasing the accumulation of drugs in target tissues. This class of drug carriers resists opsonization and elimination by the mononuclear phagocytic system (MPS or RES), thereby enabling longer blood circulation times and enhanced tissue exposure for the encapsulated drug (Lasic et al. Chem. Rev. 1995, 95, 2601-2627; Ishiwata et al., Chem. Pharm. Bull. 1995, 43, 1005-1011). Such liposomes have been shown to accumulate selectively in tumors, presumably by extravasation and capture in the neovascularized target tissues (Lasic et al., Science 1995, 267, 1275-1276; Oku et al., 1995, Biochim. Biophys. Acta, 1238, 86-90). The long-circulating liposomes enhance the pharmacokinetics and pharmacodynamics of DNA and RNA, particularly compared to conventional cationic liposomes which are known to accumulate in tissues of the MPS (Liu et al., J. Biol. Chem. 1995, 42, 24864-24870; Choi et al., International PCT Publication No. WO 96/10391; Ansell et al., International PCT Publication No. WO 96/10390; Holland et al., International PCT Publication No. WO 96/10392). Long-circulating liposomes are also likely to protect drugs from nuclease degradation to a greater extent compared to cationic liposomes, based on their ability to avoid accumulation in metabolically aggressive MPS tissues such as the liver and spleen.

Also disclosed are compositions prepared for storage or administration that include a pharmaceutically effective amount of the desired compounds in a pharmaceutically acceptable carrier or diluent. Acceptable carriers or diluents for therapeutic use are well known in the pharmaceutical art, and are described, for example, in Remington's Pharmaceutical Sciences, Mack Publishing Co. (A. R. Gennaro edit. 1985). For example, preservatives, stabilizers, dyes and flavoring agents can be provided. These include sodium benzoate, sorbic acid and esters of p-hydroxybenzoic acid. In addition, antioxidants and suspending agents can be used.

A pharmaceutically effective dose is that dose required to prevent, inhibit the occurrence, or treat (alleviate a symptom to some extent, preferably all of the symptoms) of a disease state. The pharmaceutically effective dose depends on the type of disease, the composition used, the route of administration, the type of mammal being treated, the physical characteristics of the specific mammal under consideration, concurrent medication, and other factors that those skilled in the medical arts will recognize. Generally, an amount between 0.1 mg/kg and 100 mg/kg body weight/day of active ingredients is administered dependent upon potency of the negatively charged polymer.

The nucleic acid molecules for use in the invention and formulations thereof can be administered orally, topically, parenterally, by inhalation or spray, or rectally in dosage unit formulations containing conventional non-toxic pharmaceutically acceptable carriers, adjuvants and/or vehicles. The term parenteral as used herein includes percutaneous, subcutaneous, intravascular (e.g., intravenous), intramuscular, or intrathecal injection or infusion techniques and the like. In addition, there is provided a pharmaceutical formulation comprising a nucleic acid molecule for use in the invention and a pharmaceutically acceptable carrier. One or more nucleic acid molecules for use in the invention can be present in association with one or more non-toxic pharmaceutically acceptable carriers and/or diluents and/or adjuvants, and if desired other active ingredients. The pharmaceutical compositions containing nucleic acid molecules for use in the invention can be in a form suitable for oral use, for example, as tablets, troches, lozenges, aqueous or oily suspensions, dispersible powders or granules, emulsion, hard or soft capsules, or syrups or elixirs.

Compositions intended for oral use can be prepared according to any method known to the art for the manufacture of pharmaceutical compositions and such compositions can contain one or more such sweetening agents, flavoring agents, coloring agents or preservative agents in order to provide pharmaceutically elegant and palatable preparations. Tablets contain the active ingredient in admixture with non-toxic pharmaceutically acceptable excipients that are suitable for the manufacture of tablets. These excipients can be, for example, inert diluents; such as calcium carbonate, sodium carbonate, lactose, calcium phosphate or sodium phosphate; granulating and disintegrating agents, for example, corn starch, or alginic acid; binding agents, for example starch, gelatin or acacia; and lubricating agents, for example magnesium stearate, stearic acid or talc. The tablets can be uncoated or they can be coated by known techniques. In some cases such coatings can be prepared by known techniques to delay disintegration and absorption in the gastrointestinal tract and thereby provide a sustained action over a longer period. For example, a time delay material such as glyceryl monosterate or glyceryl distearate can be employed.

Formulations for oral use can also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil.

Aqueous suspensions contain the active materials in a mixture with excipients suitable for the manufacture of aqueous suspensions. Such excipients are suspending agents, for example sodium carboxymethylcellulose, methylcellulose, hydropropylmethylcellulose, sodium alginate, polyvinylpyrrolidone, gum tragacanth and gum acacia; dispersing or wetting agents can be a naturally-occurring phosphatide, for example, lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions can also contain one or more preservatives, for example ethyl, or n-propyl p-hydroxybenzoate, one or more coloring agents, one or more flavoring agents, and one or more sweetening agents, such as sucrose or saccharin.

Oily suspensions can be formulated by suspending the active ingredients in a vegetable oil, for example arachis oil, olive oil, sesame oil or coconut oil, or in a mineral oil such as liquid paraffin. The oily suspensions can contain a thickening agent, for example beeswax, hard paraffin or cetyl alcohol. Sweetening agents and flavoring agents can be added to provide palatable oral preparations. These compositions can be preserved by the addition of an anti-oxidant such as ascorbic acid

Dispersible powders and granules suitable for preparation of an aqueous suspension by the addition of water provide the active ingredient in admixture with a dispersing or wetting agent, suspending agent and one or more preservatives. Suitable dispersing or wetting agents or suspending agents are exemplified by those already mentioned above. Additional excipients, for example sweetening, flavoring and coloring agents, can also be present.

Pharmaceutical compositions can also be in the form of oil-in-water emulsions. The oily phase can be a vegetable oil or a mineral oil or mixtures of these. Suitable emulsifying agents can be naturally-occurring gums, for example gum acacia or gum tragacanth, naturally-occurring phosphatides, for example soy bean, lecithin, and esters or partial esters derived from fatty acids and hexitol, anhydrides, for example sorbitan monooleate, and condensation products of the said partial esters with ethylene oxide, for example polyoxyethylene sorbitan monooleate. The emulsions can also contain sweetening and flavoring agents.

Syrups and elixirs can be formulated with sweetening agents, for example glycerol, propylene glycol, sorbitol, glucose or sucrose. Such formulations can also contain a demulcent, a preservative and flavoring and coloring agents. The pharmaceutical compositions can be in the form of a sterile injectable aqueous or oleaginous suspension. This suspension can be formulated according to the known art using those suitable dispersing or wetting agents and suspending agents that have been mentioned above. The sterile injectable preparation can also be a sterile injectable solution or suspension in a non-toxic parentally acceptable diluent or solvent, for example as a solution in 1,3-butanediol. Among the acceptable vehicles and solvents that can be employed are water, Ringer's solution and isotonic sodium chloride solution. In addition, sterile, fixed oils are conventionally employed as a solvent or suspending medium. For this purpose, any bland fixed oil can be employed including synthetic mono-or diglycerides. In addition, fatty acids such as oleic acid find use in the preparation of injectables.

The nucleic acid molecules for use in the invention can also be administered in the form of suppositories, e.g., for rectal administration of the drug. These compositions can be prepared by mixing the drug with a suitable non-irritating excipient that is solid at ordinary temperatures but liquid at the rectal temperature and will therefore melt in the rectum to release the drug. Such materials include cocoa butter and polyethylene glycols.

Nucleic acid molecules for use in the invention can be administered parenterally in a sterile medium. The drug, depending on the vehicle and concentration used, can either be suspended or dissolved in the vehicle. Advantageously, adjuvants such as local anesthetics, preservatives and buffering agents can be dissolved in the vehicle.

Dosage levels of the order of from about 0.1 mg to about 140 mg per kilogram of body weight per day are useful in the treatment of the above-indicated conditions (about 0.5 mg to about 7 g per subject per day). The amount of active ingredient that can be combined with the carrier materials to produce a single dosage form varies depending upon the host treated and the particular mode of administration. Dosage unit forms generally contain between from about 1 mg to about 500 mg of an active ingredient.

It is understood that the specific dose level for any particular subject depends upon a variety of factors including the activity of the specific compound employed, the age, body weight, general health, sex, diet, time of administration, route of administration, and rate of excretion, drug combination and the severity of the particular disease undergoing therapy.

For administration to non-human animals, the composition can also be added to the animal feed or drinking water. It can be convenient to formulate the animal feed and drinking water compositions so that the animal takes in a therapeutically appropriate quantity of the composition along with its diet. It can also be convenient to present the composition as a premix for addition to the feed or drinking water.

The nucleic acid molecules for use in the present invention can also be administered to a subject in combination with other therapeutic compounds to increase the overall therapeutic effect. The use of multiple compounds to treat an indication can increase the beneficial effects while reducing the presence of side effects.

### Kits

The agents for use in the present invention can be used alone or as a component of a kit having at least one of the reagents necessary to carry out the in vitro or in vivo introduction ofRNA to test samples and/or subjects. For example, preferred components of the kit include a siRNA molecule for use in the invention and a vehicle that promotes introduction of the siRNA into cells of interest as described herein (e.g., using lipids and other methods of transfection known in the art, see for example Beigelman et al, U.S. Pat. No. 6,395,713). The kit can be used for target validation, such as in determining gene function and/or activity, or in drug optimization, and in drug discovery (see for example Usman et al., U.S. Ser. No. 60/402,996). Such a kit can also include instructions to allow a user of the kit to practice the invention.

### Small Molecule and Peptide Mimetic Pharmaceutical Compositions:

The small molecule and peptide mimetics compounds which can be identified as described above and act as PirB antagonist (also referred to herein as "active compounds") can be incorporated into pharmaceutical compositions suitable for administration. Such compositions typically comprise the active compounds and a pharmaceutically acceptable carrier. As used herein the language "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. As discussed above, supplementary active compounds can also be incorporated into the compositions.

A pharmaceutical composition is formulated to be compatible with its intended route of administration. Examples of routes of administration include, but are not limited to, parenteral, e.g., intravenous, intradermal, intramuscular, intraosseous, subcutaneous, oral, intranasal, inhalation, transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™. (BASF, Parsippany, N.J.) or phosphate buffered saline (PBS). The composition preferably is sterile and should be fluid to the extent that easy syringability exists. The compositions suitably should be stable under the conditions of manufacture and storage and preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound (e.g., NeuAcα2-3(6-O-sulfo)Galβ1-4(Fucα1-3)GlcNAcβ-O-(CH₂)₃-NH-CO(CH₂)₅NH-M, where M is a hydrogen or amide linkage) in a therapeutically effective or beneficial amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. Suitable oral compositions may be e.g. enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, e.g., a gas such as hydroxyfluoroalkane (HFA), or a nebulizer. Alternatively, intranasal preparations may be comprised of dry powders with suitable propellants such as HFA.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (e.g., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially e.g. from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Pat. No. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

Toxicity and therapeutic efficacy of such compounds can be determined by standard pharmaceutical procedures in cell cultures or experimental animals, e.g., for determining the LD₅₀ (the dose lethal to 50% of the population) and the ED50 (the dose therapeutically effective in 50% of the population). The dose ratio between toxic and therapeutic effects is the therapeutic index and it can be expressed as the ratio LD₅₀/FD₅₀-Compounds which exhibit large therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

Data obtained from the cell culture assays and animal studies can be used in formulating a range of dosage for use in humans. The dosage of such compounds lies preferably within a range of circulating concentrations that include the ED50 with little or no toxicity. The dosage may vary within-this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC₅₀ (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

The pharmaceutical compositions can be included in a container, pack, or dispenser together with instructions (e.g. written) for administration, particularly such instructions for use of the active agent to treat against a disorder or disease as disclosed herein, including diseases or disorders associated with Siglec-8 expressing cells.

### EXEMPLIFICATION

This invention is further illustrated by the following examples which are provided for the purpose of illustration only and the invention should in no way be construed as being limited to these examples but rather should be construed to encompass any and all variations which become evident as a result of the teaching provided herein. The following non-limiting examples are illustrative of the invention.

### METHODS and MATERIALS

### Generation of PirBTM mice

Genomic PirB was amplified by PCR from C57/BL6 mice and inserted into the pK-11 plasmid (*40*). All constructs were verified by sequencing. A 3041 bp fragment including the exons 5,6,7,8, and 9 was amplified using primers tctgggcccgtcttctggtgaattgtatgg and gtgtgaatgtcgtgctatgg. Fragments were digested with Apa1 and cloned into the Apa1 site 5' to LoxP. A 1027 bp fragment including exons 10, 11, 12, and 13 was amplified with primers atatgtcgaccagcatgagcctgtcacac and atatgtcgacttggccctaaggtttagcac, cut with Sal I and cloned into the Sal I site, just 3' to LoxP and 5' to Frt. A 1900 bp fragment including exons 14 and 15 was amplified with primers ataccgcggataacttcgtataatgtatgctatacgaagttatgggaccatgtttcttccag, which includes a second LoxP site, and tatccgcggttaattaattgaacttagtataacagtcc, cut with Sac II, and cloned into the SacII site 3' to Frt, resulting in the construct shown in Figure S1. The vector was electroporated into 129 J1 ES cells, and positive clones were identified by PCR and by Southern hybridization with two separate probes representing the 5' and 3' ends of the construct.

Genomic DNA was extracted from founder mice and transgene was confirmed by Southern hybridization and by PCR. Mice were crossed with Cre expressing deleter strain (B6.FVB-TgN(EIIa-cre)C5379Lmgd, Jackson), expressing Cre recombinase, and mutant PirB gene with deletion of exons 10, 11, 12, and 13, was confirmed by PCR. Mutant protein was confirmed by Western blotting. Siblings from 10-15 independent matings of heterozygotes were taken to create WT and PirBTM colonies.

### In Situ Hybridization

All animal procedures were performed according to institutional guidelines and approved protocols at Harvard Medical School. Mice were anaesthetized with Halothane (Halocarbon, River Edge NJ) and euthanized by injection of 0.1 ml of Euthasol (Delmarva Laboratories). Brains were removed, placed in M1 embedding matrix (Shandon), and frozen in a dry ice/ethanol bath. In situ hybridization was performed as described (*41*). Cryostat sections (12 µm) of brain were cut, air dried, fixed for 30 min in sodium phosphate-buffered 4% paraformaldehyde, dehydrated with ethanol, and stored at -80°C. Sections were thawed, permeabilized by proteinase K treatment, acetylated, dehydrated with ethanol, and hybridized at 62°C for 12-18 hours with a riboprobe labeled with [35S] UTP (1250 Ci/mmol). The sections were then incubated with 50 µg/ml RNase A for 30 min. at 37°C and washed with a series of SSC solutions, with a high stringency wash of 0.1X SSC at 60°C for 30 min. After exposure to Kodak XAR-5 film at room temperature, sections were coated with NTB-2 emulsion and developed after 2-4 weeks. To make the PirB probes, the 3' end of PirB mRNA was amplified by RT-PCR using primer sequences tcggggaaaattcaggaa and gagaaatctctagctttattt. The T7 binding sequence taatacgactcactatagggac was added to the 3' primer in order to transcribe antisense probe, or to the 5' primer to amplify sense control probe using T7 RNA polymerase. Arc probe was transcribed from a full length Arc sequence generated by PCR.

### Antibodies

Goat anti-PirB antibodies C19 and A20 were purchased from Santa Cruz Biotechnology. C19 and A20 are specific for the cytoplasmic domain of PirB, a domain that is unique to PirB, and carries little homology to any other known mouse protein. Rat 6C1 anti-PirA/B was purchased from Pharmingen, and is specific for the extracellular domains of PirB and PirA proteins. 4G10 mouse anti-phosphotyrosine was purchased from Upstate Biotechnology. Synaptophysin monoclonal antibody SVP38 was purchased from Sigma (St. Louis). Synapsin I monoclonal antibody clone 8 was purchased from BD Transduction labs. The anti-PirB 1477 antibody was generated by immunizing a rabbit with a peptide NTEYEQAEEGQGANNQ which represents part of the PirB cytoplasmic domain. Rabbit anti-Shp-2, and mouse anti-Shp-1 were purchased from Santa Cruz Biotechnology.

### Immunostaining

Mice were anaesthetized with isofluourane, fixed by transcardial perfusion of phosphate buffered saline (PBS) followed by 4% paraformaldehyde. Brains were removed and postfixed overnight by immersion in 4% paraformaldehyde, followed by 24 hours immersion in 30% sucrose/PBS for cryoprotection. Fifty-five µm microtome sections were taken, blocked in Tris buffered saline containing 0.1% Tween and 0.5% blocking reagent (Perkin Elmer, Boston) and stained with C19 or A20 anti-PirB antibody, or control goat IgG, at 0.5 µg/ml overnight at 4°C. Signal was detected by biotinylated secondary antibody, ABC and DAB (Vector Labs, Burlingame CA). Cortical cultures were fixed for 10 minutes in 4% paraformaldehyde. Cultures were stained with 0.5-1.0 µg/ml antibody overnight at 4°C in Tris buffered saline containing 0.1% Tween and 10% horse serum, followed by fluorophore-conjugated secondary antibody, or unconjugated secondary followed by fluorophore-conjugated tertiary antibody. Actin was stained with fluorophore-conjugated phalloidin (Molecular Probes) for 15 minutes. Stained cultures were imaged under a 60X oil immersion objective.

### Soluble PirB binding

To generate the PirB-alkaline phosphatase fusion protein (PirB-AP) expression vector, the coding sequence of the extracellular domain of PirB was amplified from Image clone 4488338 (Genbank EG247984) with primers tatggcccagccggccgggtccctccctaagcctat and tataagatctcttcaggtacatatgcagtcc and inserted into the SfiI and BglII sites of the APtag-5 vector (GenHunter). As described by Flanagan et al. (2000), the resulting construct, or the APtag-5 vector alone, was transfected into 293T cells with FuGENE 6 Transfection Reagent (Roche) and soluble, His- and myc-tagged PirB-AP or AP containing conditioned media was collected approximately 6 days post-transfection. PirB-AP or AP was purified from conditioned media over a column of ProBond Resin (Invitrogen) and eluted with 100mM imidazole (Sigma). PirB-AP and AP containing fractions were identified with the soluble AP substrate p-nitro-phenyl phosphate (pNPP; Sigma), combined, and dialyzed against Neurobasal media (Gibco).

For saturation analysis of PirB-AP binding to neurons, dissociated cortical cultures were prepared from P0 CD-1 and C57B1/6 mice and grown for 5-7 DIV. Cells were incubated with varying concentrations of PirB-AP or AP (0.125 - 2 µM) for 2 hours at 37°C, washed to remove unbound AP-fusion protein, and lysed with 1% Triton X-100. Lysates were collected and endogenous AP was heat inactivated at 65°C. AP activity was measured using pNPP as substrate, reading at 405nm (*42*) in a Bio-Rad Model 680 microplate reader using the Microplate Manager Software (Version 5.2.1). Data represent background AP binding subtracted from PirB-AP binding with 8-13 measurements at each concentration from three independent experiments.

For investigation of PirB-AP binding to primary mouse embryonic fibroblasts (MEFs), fibroblasts were isolated from WWW and ß2m/Tap1 -/- embryos (*3*) at E13.5 by standard procedure (*43*). For investigation of PIRB-AP binding of MHCI in neurons, dissociated cortical neurons were prepared from P0 WWW and ß2m/Tap1 -/- mice and grown for 5-6 DIV. Equal numbers of cells from each genotype were incubated with 500 nM (MEFs) or 1 µM (neurons) AP-proteins and bound PirB-AP/AP was measured as described above using pNPP as substrate (*42*). Data represent background AP binding subtracted from PirB-AP binding with 12 (MEFs) and 15 (neurons) measurements of each genotype from three independent experiments.

To examine binding of PirB-AP to neurons in sections (*42*), unfixed brains of adult WT mice were embedded in 3% UltraPure low melting point agarose (Invitrogen) and 150-200 um sections were cut with a vibratome. Sections were incubated in PirB-AP or AP (0.5 - 1 uM ) for 16-18 hours at 4 °C, washed, and endogenous AP activity was heat inactivated at 65 °C. PirB-AP binding was visualized using the chromogenic AP substrates nitroblue tetrazolium chloride (Roche) and 5-bromo-4-chloro-3-indolylphosphate (Roche).

### Subcellular fractionation

Mouse brains were dissected and homogenized in 10 volumes of ice cold buffer (0.32 M sucrose, 10 mM HEPES (pH. 7.5), 0.1mM EDTA) using 8-10 strokes in a teflonglass homogenizer at approximately 1000 rpm. Homogenate was centrifuged at 1000 x g for 10 minutes. Pellet containing unbroken cells and debris was discarded. Supernatant was centrifuged at 10,000 x g, and pellet (P10) was collected. Supernatant was centrifuged at 100,000 x g to produce pellet (P100) and 100,000 x g supernatant (S100), which were collected and analyzed by immunoprecipitation/Western blot.

Synaptosomes were prepared as described by Nagy and Delgado-Escueta (44). One P19 CD1 mouse brain was homogenized in 10 volumes of ice cold buffer containing 0.32 M sucrose, 10 mM HEPES (pH. 7.5), 0.1mM EDTA using 8-10 strokes in teflonglass homogenizer at approximately 1000 rpm. Homogenate was centrifuged at 1000 x g for 10 minutes to give pellet P1 and supernatant S1. S1 was centrifuged at 12,000 x g for 20 minutes to produce pellet (P2) and supernatant (S2). S2 was spun at 100,000 x g to produce P100 (light membranes) and S100 (soluble fraction). P2 was resuspended in 0.5 ml sucrose buffer and applied to Percoll step gradient.

Percoll gradient was prepared by diluting SIP (9 parts Percoll (Amersham) to 1 part 2.5 M sucrose), with Medium II (0.25 M sucrose, 5mM HEPES, pH 7.2, 0.1 mM EDTA) to produce the appropriate concentrations of Percoll for the step gradient. Four ml of 16% (vol/vol) Percoll were gently overlayed with 4 ml of 10% Percoll and kept on ice. The resuspended P2 (0.5 ml) was then diluted with 4 ml of 8.5% Percoll/sucrose (7.5% Percoll final), layered onto the 10%/16% Percoll gradient and centrifuged at 15,000 x g for 20 minutes in a Sorvall SM-24 rotor. Synaptosomes were collected from the 10%/16% Percoll interface. Synaptosomes were centrifuged at 12,000 x g for 10 min, then briefly resuspended in 400 µl water (to create an isotonic shock and release synaptic vesicles) and buffered with 2 µl of 1 M HEPES pH 7.4. Synaptic vesicles were then pelleted at 25,000 x g and plasma membranes remained in the supernatant. Ten percent of the total protein of each fraction was removed for analysis of marker proteins. The remaining protein in each fraction was brought to a 1% NP-40 concentration and PirB was immunoprecipitated overnight for subsequent SDS-PAGE/Western blot analysis.

### Immunoprecipitation/Western Blots

Mouse brains were dissected and homogenized in approximately 10 volumes of ice cold buffer containing 1% NP-40, 150 mM NaCl, 50 mM Tris (pH 7.4), 1 µg/ml aprotinin, 1 µg/ml leupeptin, 1 mM phenyhnethylsulfonyl fluoride, 1mM sodium fluoride and 1mM sodium vanadate. Samples were centrifuged at 100,000 x g for 15 minutes. Immunoprecipitation was performed overnight at 4° C, or for co-immunoprecipitation, for 2 hours at 4°C. Immunoprecipitates were collected on protein G agarose (Invitrogen). Samples were separated by SDS-PAGE, transferred to PVDF or nitrocellulose membranes, probed with the appropriate antibody, and visualized with enhanced chemiluminescence (Pierce) and Kodak XAR-5 film.

### Eye enucleation

All surgical procedures were performed according to the approved animal use protocol on file at Harvard Medical School. For monocular enucleation experiments, mice were anesthetized with inhaled isofluorane. One eye was removed and eyelids were sutured with 6-0 sterile surgical silk. Opthalmic ointment (Pharmaderm) was used to prevent infection.

### Arc induction experiments

To assess functionally the ocular dominance of cortical neurons, the method of Arc mRNA induction according to Tagawa et al. was used. One eye was removed (see above) under anaesthesia; mice were revived and put in total darkness overnight. Mice were returned to a lighted environment for 30 minutes to induce Arc mRNA in the cortex driven by vision through the remaining eye. After light exposure, mice were euthanized with Halothane (Halocarbon, River Edge NJ), brains were removed, flash-frozen in M-1 mounting medium (ThermoShandon) and 16µm coronal sections were taken for in situ hybridization for Arc mRNA (23).

### Transneuronal transport of [3H] proline

To visualize the pattern of geniculocortical projections to layer 4, mice (age P35) were anesthetized with isoflurane and 200 uCi of L-[2,3,4,5-3H]-Proline (Amersham) was injected into one eye using a glass micropipette (23). After 7 days transport time, animals were anaesthetized with Halothane (Halocarbon, River Edge NJ), then euthanized by injection of 0.1 ml of Euthasol (Delmarva laboratories). Brains were removed, placed in M1 embedding matrix (Shandon), and frozen in a dry ice/ethanol bath. Coronal cryostat sections (20 µm) of brain were cut, air dried, fixed for 30 min in sodium phosphate-buffered 4% paraformaldehyde, dehydrated with ethanol, coated with NTB-2 emulsion and developed after 3 months. Images of visual cortex ipsilateral to the injected eye were acquired in darkfield optics using a Nikon Eclipse microscope. Widths of ipsilateral thalamocortical projections were measured blind to genotype by scanning (see below).

### Densitometric scans of Arc induction and transneuronal labeling in cortical layer 4

Quantitative analysis of Arc expression was performed in MATLAB (The Mathworks) by line scans in layer 4. A line along the center of the layer 4 was generated by selecting 20-100 points and then performing a cubic spline interpolation between these points. At every pixel along this line, a perpendicular line through the layer (15-30 pixels long; 1 pixel = 3.5 um) was computed and the average signal intensity of pixels along this line was measured. The resulting intensity line scan was low pass filtered (8^{th} order Butterworth), generating a curve of Arc signal intensity versus distance along layer 4. Arc signal rose to a maximum within the binocular zone. The width of the binocular zone was measured as the region around the intensity maximum in which signal intensity is greater than 2 standard deviations of the Arc background signal intensity (determined as average intensity of 15 pixels outside the binocular zone). Identical analysis techniques were used to quantify the labeling intensity of transneuronally labeled sections. The analyses were performed blind to genotype, age and manipulation; slides from different animals and manipulations were interleaved with each other and only reassembled once they were decoded.

### Anterograde labeling of retinal ganglion axons

Mice (P15) were anesthetized with isoflurane, and 1-2 µl of a 0.2% solution of cholera toxin B subunit (List Biological Laboratories) conjugated to either rhodamine (product #107) or FITC (product #106) was injected into each eye using a glass micropipette (*45*). After 24 hours, animals were euthanized with Euthasol (Delmarva laboratories). Brains were fixed by cardiac perfusion with 0.9% saline and 4% paraformaldehyde. Brains were removed and postfixed overnight in 4% paraformaldehyde at 4°C, then bathed in 30% sucrose overnight. Coronal sections (55 µm) were cut through LGN on a freezing microtome, mounted on glass slides using Vectashield (Vector Laboratories), and imaged on a Nikon Eclipse fluorescence microscope. All images were acquired such that peak intensity values were just saturating.

### Example 1: PirB Expession in Nervous Tissue

To examine if PirB is expressed in the brain, we performed in situ hybridization experiments using PirB-specific probes on mouse brain tissue sections of various age from P0 to adult. Specific mRNA signal was detected throughout the brain at all ages tested, with strong expression in cerebral cortex, hippocampus, cerebellum and olfactory bulb (Figure 1A-F). These brain regions are also know to express MHCI mRNA and protein (*1*, *2*).

Next, immunostaining for PirB protein was performed on brain sections using two different antibodies specific for the cytoplasmic domain of PirB (Figure 1G). Specific expression was detected at all ages tested, from P0 to adult (Data not shown). Protein is detected on a subset of neuronal cell bodies, and on axon pathways and neuropil.

To determine if PirB is expressed in neurons, neocortex from E17 or P0 was dissociated, cultured, and stained with three different PirB-specific antibodies, in conjunction with neuron-specific markers, including GAP-43, synapsin, PSD-95, and neurofilament (Figure 2). In these cultures, a subset of neurons, 20-50% depending on the culture, were immunostained for PirB. PirB staining is enriched in neuronal processes, where it appears as puncta, and is also present in axonal growth cones, localized to lamellipodia just behind the actin-rich leading edge (Figure 2).

To characterize brain derived PirB protein expression further, PirB protein was immunoprecipitated directly from the brain. Immunoprecipitation and subsequent Western blots were performed using different antibodies to ensure specificity. As in the immune system (*14*, *15*, *19*, *20)* brain-derived PirB exists primarily as a 130 kD glycosylated protein (Figure 3). PirB can be detected in all brain regions at all ages tested, including the optic nerve (Figure 3A). PirB from the brain is glycosylated (Figure 3B), as it is sensitive to deglycosylation by PNGaseF, which removes N-linked oligosaccharides; PirB is insensitive to Endo H, which cleaves a more restricted subset of oligosaccharides. Preparation of synaptosomes from mouse brain tissue indicates that a significant portion of PirB protein fractionates with synaptosomal plasma membranes (Figure 3C), suggesting that PirB may function at or near synapses.
A soluble recombinant fusion protein consisting of the extracellular domain of PirB fused to alkaline phosphatase (PirBAP) was used to stain cultured cortical neurons from WT mice and from mice with deleted *ß*2microglobulin and Tap1 genes (2m/Tap). These mutant mice have reduced cell surface expression of MHC Class I protein (*22*, *23*). We find that PirBAP binds specifically to WT neurons, and that this binding severely reduced in 2m/Tap -/- neurons (Figure 4), indicating that soluble PirB binds to neurons in an MHCI dependent manner.

### Example 2: Signal transduction of PirB in Nervous Tissue

The discovery of PirB expression in CNS neurons raises the question of PirB neuronal function. Therefore, we created a mutant mouse in which four exons encoding the transmembrane domain and part of PirB intracellular domain are removed, rendering PirB unable to convey signals across the plasma membrane (Figure 8). The resulting mutant mouse, which is completely viable, and mutant protein, are thus called PirBTM. To determine the effects of removing the protein's transmembrane domain and part of its intracellular signaling structure, PirB protein expression in the PirBTM mouse was examined. Figure 5A shows subcellular fractionation of wild-type and PirBTM brain, followed by immunoprecipitation of PirB and the shorter PirBTM mutant proteins, respectively. Wild-type PirB fractionates with both heavy and light membranes, with no signal detected in the soluble fraction. The mutant PirBTM protein can be detected in brain, but it is smaller and fractionates with light membranes and cytosolic fractions. Thus, loss of the transmembrane domain has altered the solubility of PirB, as expected.

We next examined the ability of mutant PirBTM to transduce signals in the brain. The cytoplasmic domain of PirB contains four immunoreceptor tyrosine-based inhibitory motifs (ITIMs). Phosphorylation of these sites recruits Shp-1 and Shp-2 phosphatases to PirB (*17-21*), and initiates a signal transduction cascade that affects the activation state immune cells. Note the PirBTM mutant lacks not only the transmembrane domain but also the ITIM most proximal to the membrane (Figure 8). When immunoprecipition was performed with anti-phosphotyrosine antibodies from wild-type and PirBTM brains, followed by anti-PirB Western blot analysis, we found that PirB was phosphorylated only in wild-type brains; no tyrosine phosphorylated PirBTM protein was detected (Figure 5B). This result was expected, as PirBTM is not a transmembrane protein and thus is unable to engage ligand, which normally leads to phosphorylation (*12*, *13*, *20*). When PirB or PirBTM is immunoprecipitated directly, followed by Western blot analysis for PirB (Figure 5C, top panel), and then followed by an anti-phosphotyrosine Western blot (bottom panel), only wild-type PirB is phosphorylated. Together, these observations suggest that PirBTM is unable to transduce signals via phosphorylation of its remaining ITIM motifs, which is the primary means by which PirB and other proteins of this class signal.

In immune cells, PirB associates with and signals primarily through Shp-1 and Shp-2 phosphatases; neuronal PirB also associates with these phosphatases (Figure 5D,E). In contrast to the immune system however, PirB appears to associate preferentially with Shp-2, as indicated by the more robust co-immunoprecipitation of PirB and Shp-2 directly from mouse brain (Figure 5D vs. 5E). This difference likely reflects the relative expression levels of Shp-1 and Shp-2 in the brain and the immune system; Shp-1 is highly expressed in immune cells but Shp-2 is more prominent in neurons (*24*, *25*). As expected, mutant PirBTM does not co-I.P. with Shp-2 (Figure 5E). Thus we conclude that signaling through PirB in the brain of PirBTM mice is abrogated.

### Example 3: Projection Pattern Development and PirB Expression

The search for neuronal MHCI receptors was inspired by the discovery that mice defective for MHCI surface expression have grossly normal brains but have abnormalities in the detailed patterns of synaptic connections in the visual system, and defects in synaptic plasticity (1). Initial observations of the PirBTM mouse also revealed no obvious phenotype. Gross histology is normal, as assessed by Niss1 staining. Because PirB is expressed on neuronal processes and growth in cones, we examined axon tracts that express PirB, such as the cortico-spinal tract and the anterior portion of the anterior commissure. Retrograde and anterograde tracing experiments indicate that, both tracts are intact and appear to have developed normal projection patterns.

### Example 4: PirB Function and Synaptic Plasticity

To examine whether PirB function is required for normal synaptic plasticity in the visual system, we studied ocular dominance development and plasticity in the primary visual cortex. This choice was motivated by the fact that PirB protein is highly expressed in cerebral cortex (Figure 1). The adult mouse visual cortex receives functional inputs from both eyes via the LGN. A restricted binocular zone receives input from both ipsilateral and contralateral eyes, whereas the rest of visual cortex consists of a large monolcular zone where neurons are driven exclusively by the contralateral eye (*26*, *27*). In development, neurons across a wide region of visual cortex receive functional input from the ipsilateral eye, but by the fourth postnatal week, this region becomes restricted, in an activity-dependent way, to the adult binocular zone. The formation of the binocular zone and the development of ocular dominance of visual cortical neurons is a reliable model for studying developmental plasticity in the mouse brain (*28*-*32*).

Ocular dominance was assessed in mouse visual cortex by means of the activity-regulated immediate-early gene Arc. If one eye is visually stimulated, Arc mRNA is rapidly induced in the visual cortex, revealing the extent of cortex functionally connected to the stimulated eye (32). This technique has provided reliable, quantitative measurements of refinement and plasticity of the ipsilateral eye representation in mouse visual cortex, and has also been validated by demonstrating ocular dominance column formation and plasticity in the cat (32). Accordingly, we examined the representation of the ipsilateral eye within the binocular zone in wild type and PirBTM mice by means of Arc induction.

In both WT and PirBTM mice at P34, stimulation of the ipsilateral eye induces Arc mRNA expression in cortical layers 2-4 and 6 in the binocular zone of the hemisphere ipsilateral to the stimulated eye (Figure 6G). This is the adult pattern of ocular dominance (32). The pattern of Arc induction in PirBTM mice at p34 appears indistinguishable from that of WT. To quantify these observations, serial line scans were made along layer 4 of visual cortex (blind to genotype) and the width of the binocular zone was measured. The width of the binocular zones of WT and PirBTM mice at P34 was identical. It is possible that the normally sized representation of the ipsilateral eye at p34 in PirBTM mice arises from an earlier widespread representation that is in fact different from normal. However, the pattern of Arc induction at P19 is also indistinguishable between PirBTM and WT (Figure 6C). Note that the width of Arc induction is larger at P19 than at P34 (figure 6B,C,D), indicating that PirB function is not needed for normal developmental restriction of the ipsilateral projection.

### Example 5: Ocular Dominance Plasticity and PirB

Activity-dependent refinement of visual system circuits occurs during a critical period when altered sensory experience can dramatically alter connectivity. Cortical ocular dominance can readily be shifted by altering the relative amounts of activity between the two eyes: this is referred to as ocular dominance plasticity. OD plasticity is more limited after the critical period (*29*, *32*-*38*). Closing or removing one eye during the critical period results in a dramatic shift in OD towards the remaining eye. This shift can be visualized directly by means of Arc inductions in cortex, ipsilateral to the remaining eye: the Arc mRNA signal expands to occupy a wider than normal zone (Figure 6E-H). Remarkably, OD plasticity in PirBTM mice was not only present, but much more robust than in WT mice at all ages tested during the critical period; P19-25, which overlaps with the peak of normal ipsilateral refinement and the peak of the critical period; P31-36, which is at the end of the critical period, and from P22-31, which spans the peak of OD plasticity. At these ages, the extent of Arc expression in layer 4 ofPirBTM mice has increased from XX% (P31-36) to as much as 50% (P19-25) of WT (Figure 6A, D, E, F, G).

The induction of Arc mRNA in the binocular zone of the visual cortex is a functional measure of ocular dominance, indicating the area in the cortex in which neurons are able to respond to visual stimuli to the ipsilateral eye. The expansion of the binocular zone in response to monocular enucleation may be due to increased intracortical connectivity, increased spread of thalamocortical axons, or both. To address this issue, we performed ME on WT and PirBTM mice from age P25-40, and visualized thalamocortical input into the cortex by transneuronal transport of 3H-proline injected into one eye (Figure 6H). These experiments demonstrate that PirBTM mice undergo greater expansion of thalamocortical axons in response to ME than in WT mice, indicating that at least some of the enhanced plasticity observed by Arc expression is likely to be due to expansion of the area innervated by thalamocortical axons. Together, the observations of enhanced plasticity at the structural level (thalamocortical axons) and function level (Arc induction in cortical neurons) indicate that PirB functions to regulate the extent of cortical OD plasticity during the critical period.

Visual stimuli originate in the eye and pass to the cortex via synaptic connections in the Lateral Geniculate Nucleus (LGN) of the thalamus. In the mouse, retinal ganglion axons that innervate the LGN undergo eye-specific segregation during the first postnatal week. The fully segregated LGN is dominated by contralateral axons, with a small region devoted to ipsilateral axons. These axons connect to eye-specific neurons in the LGN, which then project to visual cortex. Though the segregated LGN is not thought to be susceptible to experience-dependent structural plasticity, it is possible that the enhanced plasticity in the cortex of PirBTM mice is due in part to an unusually plastic LGN. Thus, we performed anterograde labeling experiments in WT and PirBTM mice to determine if ME causes the mutant LGN ipsilateral representation to expand. Figure 7 shows that indeed, the ipsilateral region of the PirBTM mice expands in response to ME, whereas in the wild-type mice, it does not, indicating the PirBTM mice do indeed have abnormally plastic LGN. These findings may account in part for the extra plasticity observed in the cortex of these mice, or PirB function may be required independently in many parts of the brain for normal plasticity.

### References

- 1.: G. S. Huh et al., Science 290, 2155-9 (Dec 15, 2000).
- 2.: R. A. Corriveau, G. S. Huh, C. J. Shatz, Neuron 21, 505-20 (Sep, 1998).
- 3.: A. Barco et al., Neuron 48, 123-37 (Oct 6, 2005).
- 4.: E. O. Long, Annu Rev Immunol 17, 875-904 (1999).
- 5.: L. L. Lanier, Annu Rev Immunol 16, 359-93 (1998).
- 6.: L. L. Lanier, Cell 92, 705-7 (Mar 20, 1998).
- 7.: A. Cerwenka, L. L. Lanier, Immunol Rev 181, 158-69 (Jun, 2001).
- 8.: A. Moretta et al., Annu Rev Immunol 14, 619-48 (1996).
- 9.: T. Takai, Adv Immunol 88, 161-92 (2005).
- 10.: T. Takai, Immunology 115, 433-40 (Aug, 2005).
- 11.: T. Takai, M. Ono, Immunol Rev 181, 215-22 (Jun, 2001).
- 12.: A. Nakamura, E. Kobayashi, T. Takai, Nat Immunol 5, 623-9 (Jun, 2004).
- 13.: S. Liang, B. Baibakov, A. Horuzsko, Eur J Immunol 32, 2418-26 (Sep, 2002).
- 14.: H. Kubagawa, P. D. Burrows, M. D. Cooper, Proc Natl Acad Sci U S A 94, 5261-6 (May 13, 1997).
- 15.: K. Hayami et al., J Biol Chem 272, 7320-7 (Mar 14, 1997).
- 16.: S. Kollnberger et al., J Immunol 173, 1699-710 (Aug 1, 2004).
- 17.: M. Blery et al., Proc Natl Acad Sci U S A 95, 2446-51 (Mar 3, 1998).
- 18.: A. Maeda et al., Oncogene 18, 2291-7 (Apr 8, 1999).
- 19.: J. F. Timms et al., Mol Cell Biol 18, 3838-50 (Jul, 1998).
- 20.: L. H. Ho, T. Uehara, C. C. Chen, H. Kubagawa, M. D. Cooper, Proc Natl Acad Sci U S A 96, 15086-90 (Dec 21, 1999).
- 21.: A. Maeda, M. Kurosaki, M. Ono, T. Takai, T. Kurosaki, J Exp Med 187, 1355-60 (Apr 20, 1998).
- 22.: J. R. Dorfman, J. Zerrahn, M. C. Coles, D. H. Raulet, J Immunol 159, 5219-25 (Dec 1, 1997).
- 23.: M. Zijlstra et al., Nature 344, 742-6 (Apr 19, 1990).
- 24.: B. G. Neel, H. Gu, L. Pao, Trends Biochem Sci 28, 284-93 (Jun, 2003).
- 25.: S. Paul, P. J. Lombroso, Cell Mol Life Sci 60, 2465-82 (Nov, 2003).
- 26.: V. S. Caviness, Jr., J Comp Neurol 164, 247-63 (Nov 15, 1975).
- 27.: U. C. Drager, J Comp Neurol 160, 269-90 (Apr 1, 1975).
- 28.: T. K. Hensch, Neurosci Res 47, 17-22 (Sep, 2003).
- 29.: N. B. Sawtell et al., Neuron 38, 977-85 (Jun 19, 2003).
- 30.: T. K. Hensch et al., Science 282, 1504-8 (Nov 20, 1998).
- 31.: J. A. Gordon, D. Cioffi, A. J. Silva, M. P. Stryker, Neuron 17, 491-9 (Sep, 1996).
- 32.: Y. Tagawa, P. O. Kanold, M. Majdan, C. J. Shatz, Nat Neurosci 8, 380-8 (Mar, 2005).
- 33.: M. Y. Frenkel, M. F. Bear, Neuron 44, 917-23 (Dec 16, 2004).
- 34.: T. A. Pham et al., Learn Mem 11, 738-47 (Nov-Dec, 2004).
- 35.: M. C. Crair, Curr Opin Neurobiol 9, 88-93 (Feb, 1999).
- 36.: L. C. Katz, J. C. Crowley, Nat Rev Neurosci 3, 34-42 (Jan, 2002).
- 37.: L. C. Katz, C. J. Shatz, Science 274, 1133-8 (Nov 15, 1996).
- 38.: D. H. Hubel, T. N. Wiesel, S. LeVay, Philos Trans R Soc Lond B Biol Sci 278, 377-409 (Apr 26, 1977).
- 39.: T. K. Hensch, Annu Rev Neurosci 27, 549-79 (2004).
- 40.: E. N. Meyers, M. Lewandoski, G. R. Martin, Nat Genet 18, 136-41 (Feb, 1998).
- 41.: D. M. Simmons, Arriza, J.L. & Swanson, L.W., J. Histotechnol. 12, 169-181 (1989).
- 42.: J. G. Flanagan, H. J. Cheng, Methods Enzymol 327, 198-210 (2000).
- 43.: B. Hogan, Beddington, R., Costantini, F., Lacy, E.,, Manipulating the mouse embryo: A laboratory manual., (Cold Spring Harbor Laboratory Press, Cold Spring Harbor, NY, ed. 2, 1994).
- 44.: A. Nagy, A. V. Delgado-Escueta, J Neurochem 43, 1114-23 (Oct, 1984).
- 45.: C. L. Torborg, M. B. Feller, J Neurosci Methods 135, 17-26 (May 30, 2004).

## Claims

1. An agent for use in treating a CNS disease or disorder in a subject, by increasing nervous system plasticity in the subject, which agent decreases the activity, signal transduction or expression of PirB in neurons of the subject, wherein the agent is selected from the group consisting of a polypeptide comprising the extracellular domain of PirB, an antibody directed to PirB, and an siRNA, ribozyme or antisense molecule capable of interfering with PirB expression.

2. An agent as claimed in claim 1, for use in treating a CNS disease or disorder in a subject selected from the group consisting of autism, dyslexia, cerebral palsy, traumatic brain injury, spinal cord injury, stroke, Alzheimer's disease, and memory disorders, by increasing nervous system plasticity in a subject, which agent modulates the activity of PirB in neurons of the subject, wherein the agent is selected from the group consisting of a polypeptide comprising the extracellular domain of PirB, and an antibody directed to PirB.

3. An agent as claimed in claim 1, for use in treating a CNS disease or disorder in a subject selected from the group consisting of autism, dyslexia, cerebral palsy, traumatic brain injury, spinal cord injury, stroke, Alzheimer's disease, and memory disorders, by increasing nervous system plasticity in a subject which agent modulates the signal transduction of PirB in neurons of the subject, wherein the agent is selected from the group consisting of a polypeptide comprising the extracellular domain of PirB, and an antibody directed to PirB.

4. An agent as claimed in claim 1, for use in treating a CNS disease or disorder in a subject selected from the group consisting of autism, dyslexia, cerebral palsy, traumatic brain injury, spinal cord injury, stroke, Alzheimer's disease, and memory disorders, by increasing nervous system plasticity in a subject, which agent decreases the expression of PirB in neurons of the subject wherein the agent is an one or more siRNA's capable of interfering with PirB expression.

5. An agent for use as claimed in claim 1, wherein the CNS disease or disorder in a subject is autism, dyslexia, cerebral palsy, traumatic brain injury, spinal cord injury, stroke, Alzheimer's disease, or memory disorders.

6. An agent for treating a visual system disorder or disease in a subject, by increasing nervous system plasticity in the subject, which agent decreases the activity, signal transduction or expression of PirB in neurons of the subject, wherein the agent is selected from the group consisting of a polypeptide comprising the extracellular domain of PirB, an antibody directed to PirB, and an siRNA, ribozyme or antisense molecule capable of interfering with PirB expression.

## Patentansprüche

1. Mittel zur Behandlung einer Erkrankung des zentralen Nervensystems (CNS) oder einer Funktionsstörung eines Probanden durch Erhöhung der Plastizität des Nervensystems des Probanden, wobei das Mittel die Aktivität, die Signalübertragung oder die Darstellung von Paarweisen Immunoglobin-ähnlichen Rezeptoren (PirB) in Nervenzellen des Probanden vermindert, wobei das Mittel aus der Gruppe ausgewählt ist, die aus Polypeptiden besteht, die ein extrazellulares Vorkommen von PirB, einen auf PirB gerichteten Antikörper und eine kurze überlagernde Ribonukleinsäure (siRNA), ein Ribozym oder ein zur Überlagerung mit einer PirB-Darstellung fähiges Antisense-Molekül umfassen.

2. Mittel nach Anspruch 1 zur Behandlung einer Erkrankung des zentralen Nervensystems (CNS) oder einer Funktionsstörung eines Probanden, ausgewählt aus der Gruppe bestehend aus Autismus, Legasthenie, infantiler Zerebralparese, traumatischen Gehirnverletzungen, Rückenmarksverletzungen, Apoplexie, Alzheimer-Krankheit und Gedächtnisstörungen, durch Erhöhung der Plastizität des Nervensystems des Probanden, wobei das Mittel die Aktivität von PirB in Nervenzellen des Probanden erhöht, wobei das Mittel aus der Gruppe ausgewählt ist, die aus Polypeptiden besteht, die ein extrazellulares Vorkommen von PirB und einen auf PirB gerichteten Antikörper umfassen.

3. Mittel nach Anspruch 1 zur Behandlung einer Erkrankung des zentralen Nervensystems (CNS) oder einer Funktionsstörung eines Probanden, ausgewählt aus der Gruppe bestehend aus Autismus, Legasthenie, infantiler Zerebralparese, traumatischen Gehirnverletzungen, Rückenmarksverletzungen, Apoplexie, Alzheimer-Krankheit und Gedächtnisstörungen, durch Erhöhung der Plastizität des Nervensystems des Probanden, wobei das Mittel die Signalübertragung von PirB in Nervenzellen des Probanden moduliert, wobei das Mittel aus der Gruppe ausgewählt ist, die aus Polypeptiden besteht, die ein extrazellulares Vorkommen von PirB und einen auf PirB gerichteten Antikörper umfassen.

4. Mittel nach Anspruch 1 zur Behandlung einer Erkrankung des zentralen Nervensystems (CNS) oder einer Funktionsstörung eines Probanden, ausgewählt aus der Gruppe bestehend aus Autismus, Legasthenie, infantiler Zerebralparese, traumatischen Gehirnverletzungen, Rückenmarksverletzungen, Apoplexie, Alzheimer-Krankheit und Gedächtnisstörungen, durch Erhöhung der Plastizität des Nervensystems des Probanden, wobei das Mittel die Darstellung von PirB in Nervenzellen des Probanden vermindert, wobei das Mittel ein oder mehrere siRNA's sind, die zur Überlagerung mit einer PirB-Darstellung geeignet sind.

5. Mittel zum Gebrauch entsprechend Anspruch 1, wobei die Erkrankung des zentralen Nervensystems (CNS) oder Funktionsstörung eines Probanden Autismus, Legasthenie, infantile Zerebralparese, traumatische Gehirnverletzungen, Rückenmarksverletzungen, Apoplexie, Alzheimer-Krankheit oder Gedächtnisstörungen ist.

6. Mittel zur Behandlung einer Störung oder Erkrankung des visuellen Systems eines Probanden durch Erhöhung der Plastizität des Nervensystems des Probanden, welches Mittel die Aktivität, die Signalübertragung oder die Expression von PirB in Nervenzellen des Probanden vermindert, wobei das Mittel aus der Gruppe ausgewählt ist, die aus Polypeptiden besteht, die ein extrazellulares Vorkommen von PirB, einen auf PirB gerichteten Antikörper und ein siRNA, ein Ribozym oder ein zur Überlagerung mit einer PirB-Darstellung fähiges Antisense-Molekül umfassen.

## Revendications

1. Agent à utiliser dans le traitement d'une maladie ou d'un trouble du SNC chez un sujet, en augmentant la plasticité du système nerveux chez le sujet, ledit agent diminuant l'activité, la transduction de signal ou l'expression du PirB dans les neurones du sujet, dans lequel l'agent est choisi dans le groupe constitué d'un polypeptide comprenant le domaine extracellulaire du PirB, un anticorps dirigé contre le PirB et un ARNsi, un ribozyme ou une molécule anti-sens capable d'interférer avec l'expression du PirB.

2. Agent selon la revendication 1, à utiliser dans le traitement d'une maladie ou d'un trouble du SNC chez un sujet choisi dans le groupe constitué de l'autisme, la dyslexie, l'infirmité motrice cérébrale, une lésion cérébrale traumatique, une lésion de la moelle épinière, un AVC, la maladie d'Alzheimer et des troubles de la mémoire, en augmentant la plasticité du système nerveux chez un sujet, ledit agent modulant l'activité du PirB dans les neurones du sujet, dans lequel l'agent est choisi dans le groupe constitué d'un polypeptide comprenant le domaine extracellulaire du PirB et un anticorps dirigé contre le PirB.

3. Agent selon la revendication 1, à utiliser dans le traitement d'une maladie ou d'un trouble du SNC chez un sujet choisi dans le groupe constitué de l'autisme, la dyslexie, l'infirmité motrice cérébrale, une lésion cérébrale traumatique, une lésion de la moelle épinière, un AVC, la maladie d'Alzheimer et des troubles de la mémoire, en augmentant la plasticité du système nerveux chez un sujet, ledit agent modulant la transduction de signal du PirB dans les neurones du sujet, dans lequel l'agent est choisi dans le groupe constitué d'un polypeptide comprenant le domaine extracellulaire du PirB et un anticorps dirigé contre le PirB.

4. Agent selon la revendication 1, à utiliser dans le traitement d'une maladie ou d'un trouble du SNC chez un sujet choisi dans le groupe constitué de l'autisme, la dyslexie, l'infirmité motrice cérébrale, une lésion cérébrale traumatique, une lésion de la moelle épinière, un AVC, la maladie d'Alzheimer et des troubles de la mémoire, en augmentant la plasticité du système nerveux chez un sujet, ledit agent diminuant l'expression du PirB dans les neurones du sujet, dans lequel l'agent est un ou plusieurs ANRsi capables d'interférer avec l'expression du PirB.

5. Agent à utiliser selon la revendication 1, dans lequel la maladie ou le trouble du SNC chez un sujet, est l'autisme, la dyslexie, l'infirmité motrice cérébrale, une lésion cérébrale traumatique, une lésion de la moelle épinière, un AVC, la maladie d'Alzheimer, ou des troubles de la mémoire.

6. Agent pour traiter un trouble ou une maladie du système visuel chez un sujet, en augmentant la plasticité du système nerveux chez le sujet, ledit agent diminuant l'activité, la transduction de signal ou l'expression du PirB dans les neurones du sujet, dans lequel l'agent est choisi dans le groupe constitué d'un polypeptide comprenant le domaine extracellulaire du PirB, un anticorps dirigé contre le PirB et un ARNsi, un ribozyme ou une molécule anti-sens capable d'interférer avec l'expression du PirB.
